(19) Europäisches Patentamt
European Patent Office
Office européen des brevets

(11) Veröffentlichungsnummer : **0 096 003 B2**

(12) # NEUE EUROPÄISCHE PATENTSCHRIFT

(45) Veröffentlichungstag der neuen Patentschrift : **15.06.94 Patentblatt 94/24**

(21) Anmeldenummer : **83810213.5**

(22) Anmeldetag : **20.05.83**

(51) Int. Cl.$^5$ : **C07D 403/12,** C07D 417/12, A01N 47/36, // C07D233/84, C07D285/125, C07D249/12

(54) **Neue Sulfonyl(thio)harnstoffe, Verfahren zu deren Herstellung und deren Verwendung als Herbizide und/oder Wachstumsregulatoren.**

(30) Priorität : **28.05.82 CH 3313/82**

(43) Veröffentlichungstag der Anmeldung : **07.12.83 Patentblatt 83/49**

(45) Bekanntmachung des Hinweises auf die Patenterteilung : **22.06.88 Patentblatt 88/25**

(45) Bekanntmachung des Hinweises auf die Entscheidung über den Einspruch : **15.06.94 Patentblatt 94/24**

(84) Benannte Vertragsstaaten : **AT BE CH DE FR GB IT LI NL**

(56) Entgegenhaltungen :
**EP-A- 41 404**
**EP-A- 0 013 480**
**EP-A- 0 030 142**
**EP-A- 0 039 239**
**EP-A- 0 044 807**
**EP-A- 0 057 546**
**EP-A- 0 064 804**
**EP-A- 0 087 780**
**EP-A- 0 095 925**
**H. Cerfontain: Mechanistic aspects m. aromatic-Sulfonatron. Interscience New York (1968),154, 155**
**R. H. Wiley: Pyrazoles, Pyrazolines, Pyrazolidines, Indazoles and condensed rings,Interscience New york (1967), 102-106, 135-136**
**C. Ferri,Reaktionen der organischen Synthese, Thieme, Stuttgart (1978), 482-487**
**R. ElderfieldHeterocyclic Compounds, Vol. 5, Wiley New York(1957), 135-136**
**Pyrazoles, Pyrazolines, Pyrazolidines, Indazoles and condensed rings, 1967, Interscience Publishers, New York, London, Sydney, title page and page 4.**
**Kirk-Othmer, Encyclopedia of Chemical Technology, 13, 1981, John Wiley & Sons, New York, Chichester, Brisbane, Toronto, title page, imprint, pages 614-618.**
**Ullmanns Encyklopädie der tecnischen Chemie, 18, 1979, Verlag Chemie, Weinheim; Deerfield Beach, Florida; Basel, Titelseite, Impressum, S. 126 bis 129.**

(56) Entgegenhaltungen :
**Enzyklopädie Naturwissenschaft und Technik, 1981, Verlag moderne chemie, Tielseite, Impressum, S. 4326**
**A.Michaelis, Annalen der Chemie 361, 251-282 (1908)**
**K. Schofield, M.R:Grimmet and B.R:T:Keene, "The Azoles", Cambridge University Press, Cambridge, 1976, Seiten 251-252**
**G.T.Morgan, I.Ackerman, J.Chem. SOC. 123, 1308-1318 (1923)**
**J.Elguero, A:R:Katritzky, P.Linda, "The Tautomerism of Heterocycles", Seiten 269-278, Academic Press, New York, 1976.**
**H.R.Christen, Grundlagen der Organischen Chemie; Verlag Sauerländer.3 Aufl.(1975) S:810**

(73) Patentinhaber : **CIBA-GEIGY AG**
**Klybeckstrasse 141**
**CH-4002 Basel (CH)**

(72) Erfinder : **Böhner, Beat, Dr.**
**Hügelweg 3**
**CH-4102 Binningen (CH)**
Erfinder : **Föry, Werner, Dr.**
**Benkenstrasse 65**
**CH-4054 Basel (CH)**
Erfinder : **Pissiotas, Georg, Dr.**
**Breslauerstrasse 8**
**D-7850 Lörrach (DE)**
Erfinder : **Schurter, Rolf, Dr.**
**Holzmattstrasse 45**
**CH-4102 Binningen (CH)**

**EP 0 096 003 B2**

## Beschreibung

Die vorliegende Erfindung umfasst eine Gruppe neuer Sulfonyl(thio)harnstoffe, die wertvolle pflanzenwachstumsregulierende Eigenschaften aufweisen und sich zur gezielten Beeinflussung des Pflanzenwuchses, zur Bekämpfung von Unkräutern z.B. in Kulturen von Nutzpflanzen und/oder zur Beeinflussung der phytotoxischen Eigenschaften anderer herbizider Substanzen eignen. Sie umfasst ferner die Herstellung dieser Sulfonyl(thio)harnstoffe, sowie landwirtschaftliche Mittel, die diese Substanzen als Wirkstoff enthalten.

Es werden hierin Verbindungen der Formel I

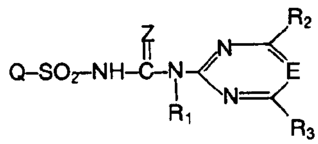

(I)

worin

$R_1$ Wasserstoff oder $C_1$-$C_5$-Alkyl;

$R_2$ und $R_3$ unabhängig voneinander Wasserstoff, $C_1$-$C_5$-Alkyl, $C_1$-$C_5$-Alkoxy, $C_1$-$C_5$-Alkylthio, $C_1$-$C_5$-Halogenalkyl, Halogen, $C_1$-$C_5$-Halogenalkoxy, $C_1$-$C_5$-Halogenalkylthio, $C_1$-$C_5$-Alkylamino, Di-$C_1$-$C_4$-alkylamino, eine Alkoxyalkylgruppe oder Alkoxyalkoxygruppe mit maximal 6 Kohlenstoffatomen bedeuten;

Z für Sauerstoff oder Schwefel steht:

E für -CH= oder -N= steht und

Q einen über ein Kohlenstoffatom gebundenen, gegebenenfalls substituierten, fünfgliedrigen, heterocyclischen Rest, ausgewählt aus der Reihe Pyrazol, Imidazol, 1,2,4-Triazol, 1,2,3-Triazol, Oxazol, Thiazol, Isooxazol, Isothiazol, Furazan, 1,2,4-Oxadiazol, 1,3,4-Oxadiazol, 1,2,3-Thiadiazol, 1,2,4-Thiadiazol und 1,3,4-Thiadiazol, bedeutet, wobei die Substituenten aus der Gruppe Halogen, Pseudohalogen, Nitro, Alkyl, Hydroxy, Haloalkyl, Alkoxy, Alkylthio, Haloalkoxy, Haloalkylthio, Amino, Alkylamino, Dialkylamino, Alkylcarbonylamino, Alkylcarbonyl, Alkoxycarbonyl, Methoxypropyl, Alkylthiocarbonyl, Carbamoyl, Alkylaminocarbonyl, Dialkylaminocarbonyl, Alkylsufinyl, Alkylsulfonyl, Alkenyloxy, Alkinyloxy und folgende gegebenenfalls durch Halogen, Nitro, Cyano, Alkyl, Alkoxy, Haloalkyl, Alkylcarbonyl, Alkoxycarbonyl oder Haloalkoxy substituierte Gruppen wie Phenyl, Phenoxy oder Phenylthio, sowie gegebenenfalls durch Halogen und/oder Alkyl substituiertes Benzyl gewählt sind; unter Einschluss ihrer Salze.

Unter Alkyl oder Alkylanteil eines anderen Substituenten sind je nach Zahl der angegebenen Kohlenstoffatome beispielsweise folgende Gruppen zu verstehen: Methyl, Äthyl, Propyl, Butyl oder Pentyl sowie ihre Isomeren wie Isopropyl, Isobutyl, tert.-Butyl, Isopentyl usw.; Halogenalkyl steht für einen ein- oder mehrfach durch Halogen substituierten Alkylrest, beispielsweise für $CF_3$, $CH_2Cl$, $CCl_3$, $CHF_2$, $CH_2J$, $C_2H_4Cl$, $C_2H_4Br$, $CH_2Br$ usw.; Halogen steht hier und im folgenden für Fluor, Chlor, Brom oder Jod, vorzugsweise für Fluor, Chlor oder Brom. Die auf dem Agrargebiet einsetzbaren Salze, sind z.B. diejenigen, die die Verbindungen der Formel I mit Aminen, Alkali- und Erdalkalimetallbasen oder mit quaternären Ammoniumbasen bilden. Hierbei sind unter Alkali- oder Erdalkalibasen insbesondere die Hydroxide von Lithium, Natrium, Kalium, Magnesium oder Calcium zu verstehen.

Beispiele für zur Salzbildung geeignete Amine sind primäre, sekundäre und tertiäre aliphatische und aromatische Amine wie Methylamin, Äthylamin, Propylamin, i-Propylamin, die vier isomere Butylamine, Dimethylamin, Diäthylamin, Diäthanolamin, Dipropylamin, Diisopropylamin, Di-n-butylamin, Pyrrolidin, Piperidin, Morpholin, Trimethylamin, Triäthylamin, Chinuclidin, Pyridin, Chinolin und i-Chinolin, insbesodere aber Äthyl-, Propyl-, Diäthyl- oder Triäthylamin, vor allem aber iso-Propylamin und Diäthanolamin.

Beispiele für quaternäre Ammoniumbasen sind im allgemeinen die Kationen von Halogenammoniumsalzen, z.B. das Tetramethylammoniumkation, das Trimethylbenzylammoniumkation, das Triäthylbenzylammoniumkation, das Tetraäthylammoniumkation, das Trimethyläthylammoniumkation, aber auch das Ammoniumkation.

Der heterocyclische Rest Q kann unsubstituiert, ein- oder mehrfach substituiert sein. Bevorzugt sind dabei Substituenten, deren aliphatischer Anteil maximal 6, vorzugsweise maximal 4 Kohlenstoffatome aufweist.

Die Verbindungen der Formel I sind bei Raumtemperatur stabile/biologisch aktive Öle, Harz oder Feststoffe, die sich durch sehr wertvolle pflanzenwachstumsregulierende, vor allem wachstumshemmende Eigenschaften auszeichnen. Sie lassen sich daher auf dem Agrarsektor oder auf verwandten Anwendungsgebieten zur gezielten Reduktion des Wachstums monocotyler oder dicotyler Pflanzen einsetzen, wobei sie je nach Art der Verwendung bzw. der Aufwandmenge selektiv herbizide bis totalherbizide Eigenschaften entfalten.

Aus der Literatur sind herbizide und pflanzenwuchsregulierende Sulfonylharnstoff-Derivate, beispielsweise aus den Europäischen Patentanmeldungen EP-A-13 480, EP-A-39 239 und EP-A-30 142 bekannt. Seit der Anmeldung der vorliegenden Erfindung sind weitere Wirkstoffe dieses Typs in den Europäischen Patentanmeldungen EP-44 807, EP-A-57 540, EP-A-64 804, EP-A-87 780 und EP-A-95 925 publiziert worden.

Zu einer bevorzugten Gruppe von Verbindungen der Formel I gehören diejenigen, worin Z Sauerstoff bedeutet und $R_1$ für Wasserstoff steht. Darüberhinaus wird diejenige Gruppe von Wirkstoffen bevorzugt, die sich aus Verbindungen der Formel I zusammensetzt, worin $R_1$ für Wasserstoff, $R_2$ und $R_3$ für $C_1$-$C_4$-Alkyl oder $C_1$-$C_4$-Alkoxy, Q und Z und E die unter Formel I angegebenen Bedeutungen haben.

Eine besonders bevorzugte Gruppe von Wirkstoffen besteht aus Verbindungen der Formel I, worin $R_1$ für Wasserstoff steht, $R_2$ und $R_3$ $C_1$-$C_3$-Alkyl oder $C_1$-$C_3$-Alkoxy, Z Sauerstoff, E Stickstoff oder CH bedeuten, Q für einen unter Formel I definierten heterocyclischen Rest steht, der gegebenenfalls durch Chlor, Brom, Fluor, Nitro, Cyano, $C_1$-$C_4$-Alkylcarbonyl, $C_1$-$C_4$-Alkoxycarbonyl, $C_1$-$C_3$-Alkoxy, $C_1$-$C_3$-Alkylthio, Methoxypropyl, $C_1$-$C_3$-Alkylsulfinyl, $C_1$-$C_3$-Alkylsulfonyl, $C_1$-$C_4$-Alkyl, Trifluormethyl, Trichlormethyl, Cyano-$C_1$-$C_4$-alkyl, $C_2$-$C_4$-Alkenyloxy, $C_3$-$C_4$-Alkinyloxy oder N-$C_1$-$C_4$-Alkylcarbonylamino substituiert ist.

Folgende Einzelsubstanz ist besonders bevorzugt:
N-[1-(3-Methoxy-1-propyl)-imidazol-2-ylsulfonyl]-N'-(4-methoxy-6-methylpyrimidin-2-yl)-harnstoff.

Die Wirkstoffe der Formel I können in an sich bekannter Weise in einem reaktionsinerten, organischen Lösungsmittel oder Lösungsmittelgemisch hergestellt werden.

Man kann dabei im einzelnen folgendermassen vorgehen und Verbindungen der Formel I, gemäss Gleichung [1],

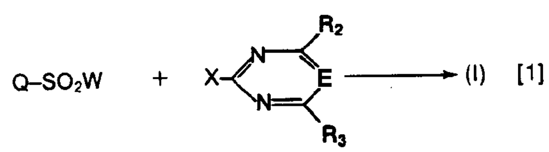

(II) (III)

dadurch herstellen, dass man ein Phenylsulfonylderivat II mit einer Verbindung der Formel III umsetzt, wobei X und W für die Gruppen $-NH_2$, -N = C = Z oder $-NR_1$-CZ-OR stehen; die Substituenten $R_1$, $R_2$, $R_3$, Q, Z und E die unter Formel I angegebenen Bedeutungen haben, R für einen aliphatischen oder aromatischen Rest, vorzugsweise für $C_1$-$C_4$-Alkyl, Phenyl oder Benzyl steht; mit der Massgabe, dass die Reaktionspartner der Formeln II und III stets so gewählt werden, dass eine Aminofunktion entweder mit einer Isocyanato- bzw. Isothiocyanatofunktion oder mit der [$-NR_1$-CZ-OR)-gruppe zur Reaktion gelangt.

Das Verfahren wird vorteilhafterweise in Gegenwart einer Base durchgeführt. In manchen Fällen kann es von Vorteil sein, wenn man unter Schutzgasatmosphäre z.B. unter Stickstoff arbeitet.

Die Herstellungsverfahren sind Bestandteil vorliegender Erfindung.

Die erhaltenen Produkte der Formel I können gewünschtenfalls durch Reaktion mit Aminen, Alkalimetall- oder Erdalkalimetallhydroxiden oder quaternären Ammoniumbasen in basische, landwirtschaftlich verwendbare Additionssalze übergeführt werden. Dieses geschieht beispielsweise durch Umsetzen mit der äquimolaren Menge Base und Verdampfen des Lösungsmittels. Solche Umsetzungen wie auch die Herstellung einzelner Ausgangsstoffe der Formel III sind bekannt und beispielsweise in den US Patentschriften Nr. 3 384 757 und 3 410 887 beschrieben.

Die Ausgangsverbindungen der Formel III und die der Formel II, in der W für $NH_2$ oder -N = C = O steht, sind bekannt oder werden nach an sich bekannten Methoden hergestellt.

Die Ausgangsverbindungen der Formel II, in denen W für $-NR_1$-CZ-OR steht, sind neu und sind speziell für die Synthese der Verbindungen der Formel I entwickelt worden. Sie bilden daher einen weiteren Aspekt der vorliegenden Erfindung. Ihre Herstellung erfolgt nach an sich bekannten Methoden.

So werden die Azolyl-sulfonamide der Formel II durch Umsetzung der entsprechenden Sulfonylchloride mit Ammoniak oder Ammoniumhydroxid-Lösungen gewonnen, siehe Gazz. chim. ital. 70, 1 (1940); US-PS 2 603 649; GB-PS 710 614; J. org. Chem.19, 894 (1954); Arch. Pharm. 278, 437 (1940); FR-PS 1 002 201.

Die Isocyanate der Formeln II und III können durch Umsetzung der zugrundeliegenden Amino-Verbindungen mit Phosgen in Anwesenheit von Butylisocyanat in einem chlorierten Kohlenwasserstoff als Lösungsmittel bei Rückflusstemperatur erhalten werden. Siehe dazu « Neuere Methoden der präparativen organischen Chemie » Band VI, 211-229, Verlag Chemie, Weinheim, 1970. Bei Verwendung von Thiophosgen gelangt man auf analoge Weise zu Isothiocyananten II und III.

Die Isothiocyanate der Formel II werden darüberhinaus auch durch Behandlung der Sulfonamide II mit Schwefelkohlenstoff und Kaliumhydroxid und anschliessender Umsetzung des Dikaliumsalzes mit Phosgen

erhalten, siehe Arch. Pharm. 299, 174 (1966).

Verbindungen der Formeln II und III, worin W und X für die Gruppe $-NR_1-CZ-OR$ steht, können aus den zugrundeliegenden Aminoverbindungen durch Reaktion mit einem Halogen(thio)ameisensäureester der Formel IV

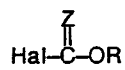

(IV)

oder einem (Thio)Kohlensäurediester der Formel V

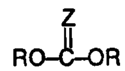

(V)

hergestellt werden; hierbei steht Hal für ein Halogen, vorzugsweise Chlor oder Brom, Z steht für Sauerstoff oder Schwefel und R für einen aliphatischen oder aromatischen Rest, vorzugsweise für $C_1$-$C_4$-Alkyl, Phenyl oder Benzyl. Diese Reaktion wird bevorzugt in Gegenwart einer Base durchgeführt.

Die als Ausgangsverbindungen der Formel III benützten Amine sind bekannt oder können nach an sich bekannten Methoden zur Synthese heterocyclischer Amine hergestellt werden. Wir verweisen dazu auf folgende Literaturstellen: « The Chemistry of Heterocyclic Compounds », Band XVI Interscience Publishers, New York, London, worin sowohl Synthesen für 2-Aminopyrimidine wie auch für 2-Amino-1,2,5-triazine zu finden sind.

Diese Umsetzungen werden vorteilhafterweise in aprotischen, inerten, organischen Lösungsmitteln vorgenommen wie Methylenchlorid, Tetrahydrofuran, Acetonitril, Dioxan, Toluol.

Die Reaktionstemperaturen liegen vorzugsweise zwischen - 20° und + 120 °C. Die Umsetzungen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base oder Isocyanat als Reaktionskatalysator beschleunigt werden.

Die Wirkstoffe der Formel I sind stabile Verbindungen. Sie sind für Warmblüter wenig giftig.

Bei der Herstellung der Verbindungen der Formel I nach Gleichung [1] können in gewissen Fällen durch Addition einer Verbindung der Formel II an ein Ring-Stickstoffatom einer Verbindung der Formel III isomere Reaktionsprodukte der Formeln Ia bzw. Ib isoliert werden:

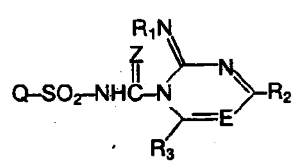

(Ia)

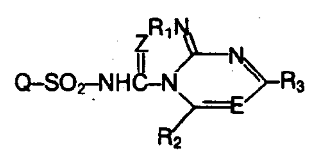

(Ib)

Hierbei haben $R_1$, $R_2$, $R_3$, Q, Z und E die unter Formel I angegebenen Bedeutungen. Auch diese isomeren Produkte Ia und Ib beeinflussen das Wachstum von Pflanzen. Die vorliegende Erfindung umfasst somit alle agrarchemisch verwendbaren Produkte und Produktgemische, die bei der Umsetzung der Verbindungen der Formel II mit III gebildet werden. Sulfonylharnstoffe mit zwei oder drei Heteroatomen im Molekülteil Q sind ausser aus den nach dem Anmeldedatum publizierten Europäischen Patentanmeldungen EP-A-87 780 und 95 925 noch nicht bekannt.

Die neuen Wirkstoffe der Formel I weisen selektiv-herbizide Eigenschaften auf und eignen sich daher zur Verwendung in Kulturen von Nutzpflanzen, beispielsweise Getreide, Soja und Baumwolle.

Einige Verbindungen der Formel I zeigen translozierende Eigenschaften, d.h. sie werden an der behandelten Stelle der Pflanze (Blatt, Stiel, Wurzel etc.) aufgenommen und an andere Stellen transportiert, wo sie

ihre Wirkung entfalten. Sie folgen hierbei nicht nur der Richtung des Nährstofftransportes von den Wurzeln in die Blätter, sondern gelangen auch umgekehrt von den Blättern zu den Wurzeln. So gelingt es mit Hilfe dieser translozierenden Eigenschaft z.B. durch eine Oberflächenbehandlung perennierende Unkräuter bis in die Wurzeln zu vernichten. Darüberhinaus wirken die Wirkstoffe der Formel I im Vergleich zu herkömmlichen Herbiziden bereits in sehr geringen Aufwandmengen.

Ferner können Verbindungen der Formel I die phytotoxischen Eigenschaften anderer herbizider Substanzen gegenüber gewissen Schadpflanzen verstärken und gegenüber einigen Kulturpflanzen vermindern. Dies kann zu einer Verringerung der gesamten Aufwandmenge an herbiziden Wirkstoffen und damit zu einer geringeren Umweltbelastung führen.

Werden die Aufwandmengen der erfindungsgemässen Wirkstoffe weiter gesenkt, so treten vor allem die wachstumsregulierenden Eigenschaften in den Vordergrund, dabei greifen die Verbindungen der Formel I gezielt in den Metabolismus der Pflanzen ein und führen insbesondere zu einer Reduktion des vegetativen Wachstums, oft zugunsten des generativen Wachstums. Dieser gezielte Eingriff in die physiologischen Vorgänge der Pflanzenentwicklung macht die Wirkstoffe der Formel I für verschiedene Zwecke verwendbar, insbesondere für solche, die mit der Arbeitseinsparung bei Massnahmen an Pflanzenkulturen oder der Ertragssteigerung im Zusammenhang stehen.

Hierin offenbarte Verbindungen der Formel I können als Herbizide und/oder als Wachstumsregulatoren eingesetzt werden. Nach bisherigen Erfahrungen gilt für die Applikation von Wachstumsregulatoren, dass die Aktivsubstanzen eine oder mehrere unterschiedliche Wirkungen bei den Pflanzen hervorrufen können. Diese verschiedenartigen Wirkungen hängen im wesentlichen vom Zeitpunkt der Anwendung, d. h. vom Entwicklungsstadium des Samens oder der Pflanze, der Art der Applikation sowie insbesondere von den angewendeten Konzentrationen ab. Derartige Effekte sind aber wiederum je nach Pflanzenart verschieden. Die Applikation von Verbindungen der Formel I eröffnet somit die Möglichkeit das Wachstum von Pflanzen in gewünschter Weise zu beeinflussen.

Mit den neuen Wirkstoffen der Formel I bzw. mit den neuen Mitteln wird das vegetative Pflanzenwachstum gedämpft. Durch diese Beeinflussung des Wachstums können die erfindungsgemäss verwendeten Wirkstoffe die Ernteausbeute von Pflanzen wesentlich erhöhen. So erfahren etwa Sojapflanzen und andere Leguminosen wie Bohnen, Erbsen oder Linsen eine Reduktion des vegetativen zugunsten des generativen Wachstums, wodurch eine unmittelbare Ertragssteigerung erzielt wird. Auch bei weiteren Pflanzenarten, z.B. bei Reben, Getreidesorten, Gräsern und Zierpflanzen wird das vegetative Wachstum in gewünschter Weise gehemmt. Daneben beobachtet man bei den behandelten Pflanzen eine deutliche Stärkung des Stützgewebes.

Eine im Vordergrund stehende Art der Pflanzenbeeinflussung beruht auf der besonderen Eigenschaft der Verbindungen der Formel I, bei bestimmten Pflanzen, insbesondere bei Getreidekulturen, eine gezielte Wuchshemmung hervorzurufen, die bei unveränderter Ertragsleistung die Knickfestigkeit der Pflanzen wesentlich erhöht. Daraus ergibt sich eine wirtschaftlich sehr interessante Methode des Schutzes von Pflanzenkulturen vor Lagerung durch Sturm oder Unwetter. Darüberhinaus gestattet eine gezielte Hemmung des vegetativen Wachstums bei vielen Kulturpflanzen eine dichtere Anpflanzung der Kulturen; dies führt bei gleichem Fruchtansatz und gleicher Anbaufläche zu einer deutlichen Ertragssteigerung. Der Einsatz von Wuchshemmern bewirkt auch eine effektivere Nutzung der Nährstoffe, die nunmehr in verstärktem Masse der Blüten und Fruchtbildung zugute kommen. Auf diese Weise lassen sich höhere Ernteerträge bei gleichzeitig kleinerem Abfallanteil an vegetativen Pflanzenresten [z.B. Stroh, Kartoffelkraut, Rübenblätter] erzielen.

Hervorzuheben ist auch die Möglichkeit, mit den erfindungsgemässen Stoffen bzw. Mitteln bei verschiedenen Pflanzenarten, insbesondere bei Tabakpflanzen eine Hemmung des unerwünschten Geiztriebwuchses zu erreichen, wenn der Haupttrieb kurz vor der Blüte zum Zwecke der erstrebten Wuchsvergrösserung der Blätter abgeschnitten worden ist.

Die Erfindung betrifft somit eine herbizide und/oder wachstumsregulierende Verwendung der Verbindungen der Formel I. Eingeschlossen ist auch ein Verfahren zur pre- und post-emergen Unkrautbekämpfung, zur Hemmung des Pflanzenwuchses von monokotylen und dicotylen Pflanzen, insbesondere von Gräsern, Getreidesorten und Tabakgeiztrieben sowie zur Steigerung des Ernteertrages bei Leguminosen.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Öl- oder Stearinsäure, oder von natürlichen Fettsäuregemischen, die z.B. aus Kokosnuss- oder Talgöl gewonnen werden können, genannt. Ferner sind auch die Fettsäuremethyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Äthylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylenpolyäthylenoxidaddukte, Tributylphenoxypolyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl oder niedrige Hydroxyalkylreste aufweisen. Die Salze liegen vorzugsweise als Halogenide, Methylsulfate oder Äthylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

« Mc Cutcheon's Detergents and Emulsifiers Annual » MC Publishing Corp., Ridgewood, New Jersey,

1979. Sisley and Wood, « Encyclopedia of Surface Active Agents » Chemical Publishing Co., Inc. New York, 1964.

Die pestiziden Zubereitungen enthalten in der Regel 0,1 bis 95%, insbesondere 0,1 bis 80%, Wirkstoff der Formel I, 1 bis 99,9% eines festen oder flüssigen Zusatzstoffes und 0 bis 25%, insbesondere 0,1 bis 25% eines Tensides.

Insbesondere setzen sich bevorzugte Formulierungen folgendermassen zusammen: (% = Gewichtsprozent).

Emulgierbare Konzentrate

| | |
|---|---|
| Aktiver Wirkstoff: | 1 bis 20%, bevorzugt 5 bis 10% |
| oberflächenaktives Mittel: | 5 bis 30%, vorzugsweise 10 bis 20% |
| flüssiges Trägermittel: | 50 bis 94%, vorzugsweise 70 bis 85%. |

Stäube

| | |
|---|---|
| Aktiver Wirkstoff: | 0,1 bis 10%, vorzugsweise 0,1 bis 1% |
| festes Trägermittel: | 99,9 bis 90%, vorzugsweise 99,9 bis 99%. |

Suspension-Konzentrate

| | |
|---|---|
| Aktiver Wirkstoff: | 5 bis 75%, vorzugsweise 10 bis 50% |
| Wasser: | 94 bis 25%, vorzugsweise 90 bis 30% |
| oberflächenaktives Mittel: | 1 bis 40%, vorzugsweise 2 bis 30%. |

Benetzbare Pulver

| | |
|---|---|
| Aktiver Wirkstoff: | 0,5 bis 90%, vorzugsweise 1 bis 80% |
| oberflächenaktives Mittel: | 0,5 bis 20%, vorzugsweise 1 bis 15% |
| festes Trägermittel: | 5 bis 95%, vorzugsweise 15 bis 90%. |

Granulate

| | |
|---|---|
| Aktiver Wirkstoff: | 0,5 bis 30%, vorzugsweise 3 bis 15% |
| festes Trägermittel: | 99,5 bis 70%, vorzugsweise 97 bis 85%. |

Während als Handelsware eher konzentrierte Mittel bevorzugt werden, verwendet der Endverbraucher in der Regel verdünnte Mittel. Die Anwendungsformen können bis hinab zu 0,001% an Wirkstoff verdünnt werden. Die Aufwandmengen betragen in der Regel 0,01 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die Erfindung umfasst auch derartige landwirtschaftlich verwendbare Mittel, die als mindestens eine Aktivsubstanz eine Verbindung der Formel I enthalten.

Darüberhinaus schliesst die vorliegende Erfindung auch die Herstellung solcher Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

In den nachfolgenden Beispielen sind Temperaturen in Celsiusgraden angegeben, Prozentangaben und Teile beziehen sich auf das Gewicht.

Herstellungsbeispiele:

Beispiel 1:

a) Herstellung des Zwischenproduktes

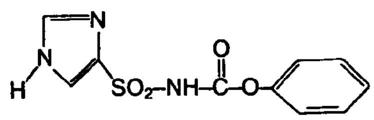

N-(4-Imidazolylsulfonyl)phenylcarbamat

Zu einer Lösung von 7,35 g 4-Imidazolylsulfonamid in 100 ml Dimethylformamid werden unter Stickstoffatmosphäre bei Raumtemperatur 2,18 g einer 55%igen Natriumhydriddispersion in Öl portionenweise während 15 Minuten zugegeben und weitere 20 Minuten reagieren gelassen.

Zu dieser Lösung werden bei Raumtemperatur 11,24 g Diphenylcarbonat, gelöst in 40 ml DMF während 30 Minuten zugetropft. Das Reaktionsgemisch wird weitere 30 Minuten ausgerührt, auf ein Gemisch von 400 ml Eis und 50 ml 2n HCL gegossen, der gebildete Niederschlag in 300 ml Essigester aufgenommen, 2 mal mit Essigester und Eiswasser nachextrahiert, getrocknet und eingedampft. Man erhält das gewünschte Carbamat in quantitativer Ausbeute. Smp.: 198-200°.

b) Herstellung des Endproduktes

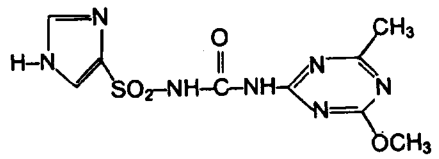

N-(4-Imidazolylsulfonyl)-N′-(4-methyl-6-methoxy-1,3,5-triazin-2-yl)-harnstoff

Eine Suspension von 13,35 g N-(4-Imidazolylsulfonyl)phenylcarbamat und 7 g 2-Amino-4-methyl-6-methoxy-1,3,5-triazin in 140 ml absolutem Dioxan wird während 75 Minuten bei Rückflusstemperatur gerührt. Das Reaktionsgemisch wird heiss filtriert, das gekühlte Filtrat mit 8,4 ml Isopropylamin versetzt. Das Lösungsmittel abdekantiert und das semikristalline Isopropylammoniumsalz dreimal mit Essigester gewaschen. Das Salz wird kalt in 200 ml Wasser gelöst, filtriert, das Filtrat mit 2n Salzsäure auf pH 1 gebracht und filtriert. Der Filterrückstand wird mit kaltem Wasser gewaschen und getrocknet.
Man erhält 5,6 g des gewünschten Sulfonylharnstoffs. Smp. ab 147° unter Zersetzung.

Beispiel 2:

a) Herstellung des Ausgangsproduktes

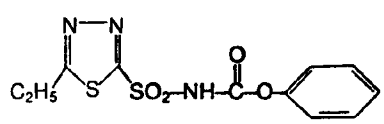

N-(5-Äthyl-1,3,4-thiadiazolyl-2-sulfonyl)-phenylcarbamat

Eine Lösung von 9,7 g 5-Äthyl-1,3,4-thiadiazol-2-sulfonamid in 100 ml absolutem Dimethylformamid wird unter Rühren unter Stickstoffatmosphäre bei Raumtemperatur portionsweise mit 2,2 g Natriumhydrid versetzt. Nach 15-minütigem Rühren gibt man tropfenweise eine Lösung von 11,2 g Diphenylcarbonat in 30 ml Dimethylformamid dazu. Das Ganze wird noch drei Stunden weitergerührt und mit 50 ml HCl 2N/500 ml Wasser behandelt. Die ausgefallenen Kristalle werden nach dem Abnutschen mit Wasser gewaschen und getrocknet.

Man erhält 12,8 g einer Substanz der obigen Formel mit dem Schmelzpunkt 142°C.

b) Herstellung des Endproduktes

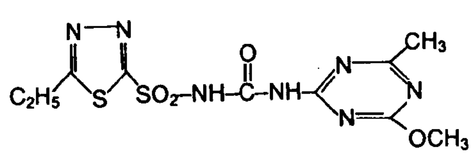

N-(5-Äthyl-1,3,4-thiadiazolyl-2-sulfonyl)-N′-(4-methyl-6-methoxy-1,3,5-triazin-2-yl)-harnstoff

Ein Gemisch von 12,8 g des unter a) beschriebenen Produkts und 5,8 g 2-Amino-4-methoxy-6-methyl-1,3,5-triazin in 100 ml absolutem Dioxan wird 1 Stunde lang am Rückfluss gekocht. Nach dem Eindampfen wird der Rückstand mit Hexan versetzt.

Man erhält 2,9 g einer weissen Substanz der obigen Formel mit dem Schmelzpunkt 60°C.

Beispiel 3:

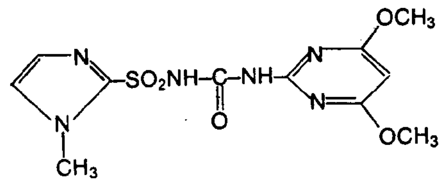

N-(1-Methylimidazol-2-ylsulfonyl)-N'-(4,6-dimethoxypyrimidin-2-yl)-harnstoff

2,4 g 1-Methylimidazol-2-yl-sulfonamid werden in 50 ml absolutem Dioxan gelöst und mit 2,25 g 1,8-Diazabicyclo[5.4.0]undec-7-en versetzt. Dann werden 4,2 g 4,6-Dimethoxy-2-phenoxycarbonylamino-pyrimidin portionenweise innerhalb von 30 Minuten bei 20-25°C zugesetzt. Nach 4 Std. wird das Reaktionsgemisch mit Eiswasser aufgenommen und mit 2-N HCl angesäuert. Dabei fällt ein Niederschlag aus. Man setzt 50 ml Essigester zu, filtriert, wäscht den Niederschlag mit Wasser und Essigester nach und trocknet ihn anschliessend.

Man erhält so 4,0 g (78%) farbloses, kristallines Produkt vom Smp. 189°C (Zers.).

Auf analoge Weise können die in den folgenden Tabellen aufgelisteten Zwischen- und Endprodukte hergestellt werden. Die in Tabelle 1 definierten Heterocyclen Q werden in den übrigen Tabellen mit gleicher abgekürzter Bedeutung verwendet.

Tabelle 1:

$Q-SO_2-NH_2$

| Nr. | Q | Abkürzung | Phys. Daten |
|---|---|---|---|
| 1.1 | 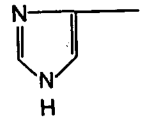 | $Q_1$ | |
| 1.2 | 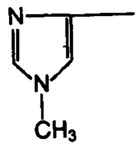 | $Q_2$ | |
| 1.3 | 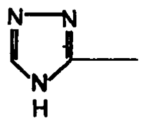 | $Q_3$ | |
| 1.4 | 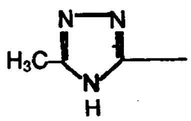 | $Q_4$ | |
| 1.5 | 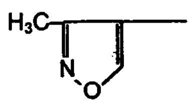 | $Q_5$ | |
| 1.6 | 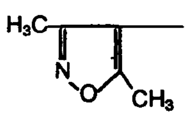 | $Q_6$ | |
| 1.7 | 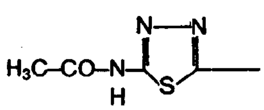 | $Q_7$ | |
| 1.8 | 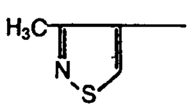 | $Q_8$ | |
| 1.9 | 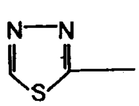 | $Q_9$ | |

Tabelle 1: (Fortsetzung)

| Nr. | Q | Abkürzung | Phys. Daten |
|---|---|---|---|
| 1.10 | 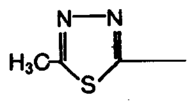 | $Q_{10}$ | |
| 1.11 | 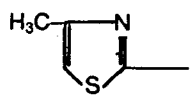 | $Q_{11}$ | |
| 1.12 | 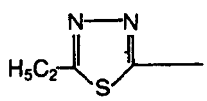 | $Q_{12}$ | |
| 1.13 | 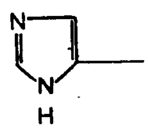 | $Q_{13}$ | |
| 1.14 | 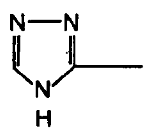 | $Q_{14}$ | |
| 1.15 | 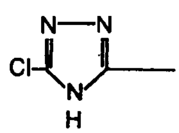 | $Q_{15}$ | |
| 1.16 | 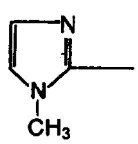 | $Q_{16}$ | Smp. 142–145 °C |
| 1.17 | 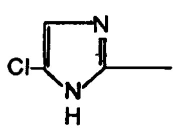 | $Q_{17}$ | |

Tabelle 1: (Fortsetzung)

| Nr. | Q | Abkürzung | Phys. Daten |
|---|---|---|---|
| 1.18 | 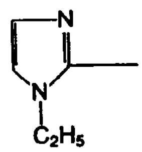 | $Q_{18}$ | |
| 1.19 | 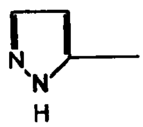 | $Q_{19}$ | |
| 1.20 | 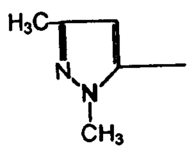 | $Q_{20}$ | |
| 1.21 | 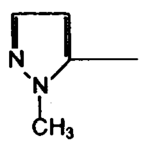 | $Q_{21}$ | |
| 1.22 | 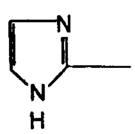 | $Q_{22}$ | |
| 1.23 | 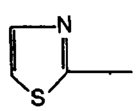 | $Q_{23}$ | |
| 1.24 | 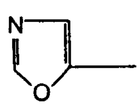 | $Q_{24}$ | |
| 1.25 | 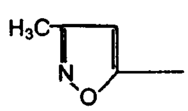 | $Q_{25}$ | |
| 1.26 | 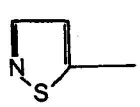 | $Q_{26}$ | |

Tabelle 1: (Fortsetzung)

| Nr. | Q | Abkürzung | Phys. Daten |
|---|---|---|---|
| 1.27 | 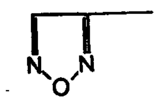 | $Q_{27}$ | |
| 1.28 | 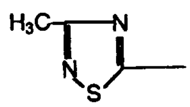 | $Q_{28}$ | |
| 1.29 | 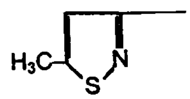 | $Q_{29}$ | |
| 1.30 | 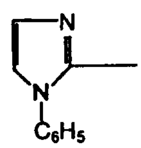 | $Q_{30}$ | Smp. 168–169 °C |
| 1.32 | 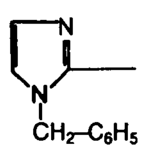 | $Q_{32}$ | Smp. 114 °C |
| 1.33 | 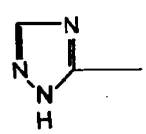 | $Q_{33}$ | |
| 1.34 | 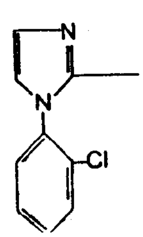 | $Q_{34}$ | Smp. 179 °C |
| 1.35 | 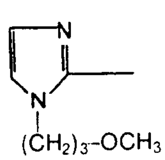 | $Q_{35}$ | Smp. 92 °C |

Tabelle 1: (Fortsetzung)

| Nr. | Q | Abkürzung | Phys. Daten |
|---|---|---|---|
| 1.36 | 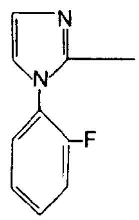 | $Q_{36}$ | Smp. 182 °C |
| 1.37 | 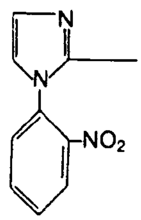 | $Q_{37}$ | Smp. 152 °C |
| 1.38 | 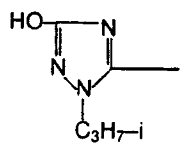 | $Q_{38}$ | Smp. 183 °C (Zers.) |
| 1.39 | 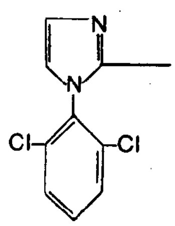 | $Q_{39}$ | Smp. 223–225 °C |
| 1.40 | 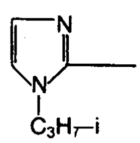 | $Q_{40}$ | |
| 1.41 | 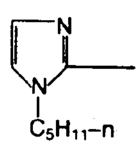 | $Q_{41}$ | |
| 1.42 | 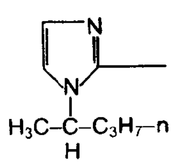 | $Q_{42}$ | |
| 1.43 | 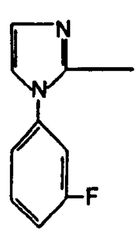 | $Q_{43}$ | |

Tabelle 1: (Fortsetzung)

| Nr. | Q | Abkürzung | Phys. Daten |
|---|---|---|---|
| 1.44 | 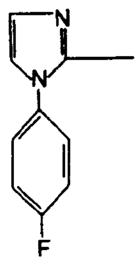 | $Q_{44}$ | |
| 1.45 | 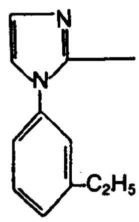 | $Q_{45}$ | |
| 1.46 | 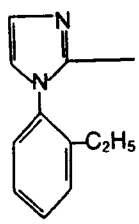 | $Q_{46}$ | |
| 1.47 | 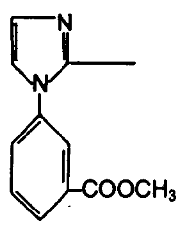 | $Q_{47}$ | |
| 1.48 | 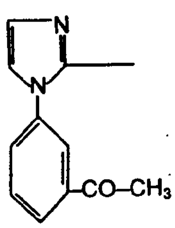 | $Q_{48}$ | |
| 1.49 | 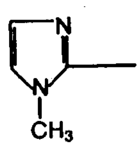 | $Q_{49}$ | |
| 1.50 | 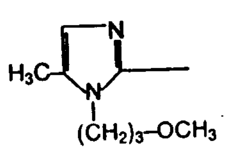 | $Q_{50}$ | |

Tabelle 1: (Fortsetzung)

| Nr. | Q | Abkürzung | Phys. Daten |
|---|---|---|---|
| 1.51 | 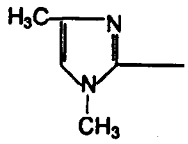 | $Q_{51}$ | |
| 1.52 | 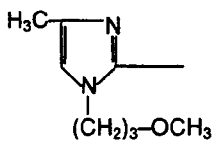 | $Q_{52}$ | |
| 1.53 | 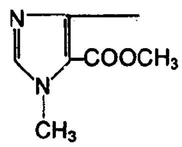 | $Q_{53}$ | |
| 1.54 | 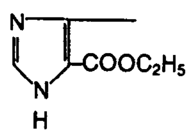 | $Q_{54}$ | |
| 1.55 | 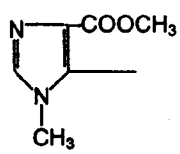 | $Q_{55}$ | |
| 1.56 | 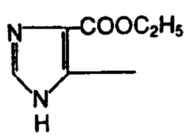 | $Q_{56}$ | |
| 1.57 | 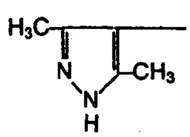 | $Q_{57}$ | |
| 1.58 | 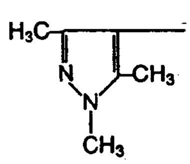 | $Q_{58}$ | |
| 1.59 | 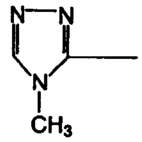 | $Q_{59}$ | |

Tabelle 1: (Fortsetzung)

| Nr. | Q | Abkürzung | Phys. Daten |
|---|---|---|---|
| 1.60 | 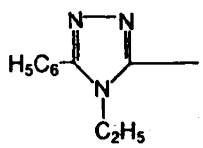 | $Q_{60}$ | |
| 1.61 | 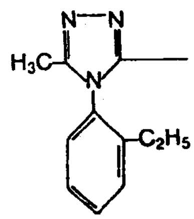 | $Q_{61}$ | |
| 1.62 | 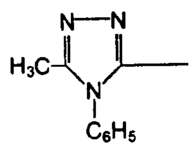 | $Q_{62}$ | |
| 1.63 | 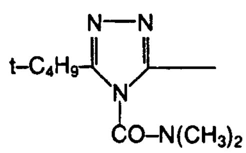 | $Q_{63}$ | |

Tabelle 2:

Q–SO$_2$–NH–CO–OR

| Nr. | Q | R | Phys. Daten |
|---|---|---|---|
| 2.1 | $Q_1$ | $C_6H_5$ | Smp. 198–200 °C |
| 2.2 | $Q_2$ | $C_6H_5$ | |
| 2.3 | $Q_3$ | $C_6H_5$ | |
| 2.4 | $Q_4$ | $C_6H_5$ | |
| 2.5 | $Q_5$ | $C_6H_5$ | |
| 2.6 | $Q_6$ | $C_6H_5$ | |
| 2.7 | $Q_7$ | $C_6H_5$ | |
| 2.8 | $Q_8$ | $C_6H_5$ | |
| 2.9 | $Q_9$ | $C_6H_5$ | |
| 2.10 | $Q_{10}$ | $C_6H_5$ | |
| 2.11 | $Q_{11}$ | $C_6H_5$ | |
| 2.12 | $Q_{12}$ | $C_6H_5$ | Smp. 142 °C |
| 2.13 | $Q_{13}$ | $C_6H_5$ | |
| 2.14 | $Q_{14}$ | $C_6H_5$ | |
| 2.15 | $Q_{15}$ | $C_6H_5$ | |
| 2.16 | $Q_{16}$ | $C_6H_5$ | |
| 2.17 | $Q_{17}$ | $C_6H_5$ | |
| 2.18 | $Q_{18}$ | $C_6H_5$ | |
| 2.19 | $Q_{19}$ | $C_6H_5$ | |
| 2.20 | $Q_{20}$ | $C_6H_5$ | |
| 2.21 | $Q_{21}$ | $C_6H_5$ | |
| 2.22 | $Q_{22}$ | $C_6H_5$ | |
| 2.23 | $Q_{23}$ | $C_6H_5$ | |
| 2.24 | $Q_{24}$ | $C_6H_5$ | |
| 2.25 | $Q_{25}$ | $C_6H_5$ | |
| 2.26 | $Q_{26}$ | $C_6H_5$ | |

Tabelle 2: (Fortsetzung)

| Nr. | Q | R | Phys. Daten |
|---|---|---|---|
| 2.27 | $Q_{27}$ | $C_6H_5$ | |
| 2.28 | $Q_{28}$ | $C_6H_5$ | |
| 2.29 | $Q_{29}$ | $C_6H_5$ | |
| 2.30 | $Q_{30}$ | $C_6H_5$ | |
| 2.32 | $Q_{32}$ | $C_6H_5$ | |
| 2.33 | $Q_{33}$ | $C_6H_5$ | |
| 2.34 | $Q_{34}$ | $C_6H_5$ | |
| 2.35 | $Q_{35}$ | $C_6H_5$ | |
| 2.36 | $Q_{36}$ | $C_6H_5$ | |
| 2.37 | $Q_{37}$ | $C_6H_5$ | |
| 2.38 | $Q_{38}$ | $C_6H_5$ | |
| 2.39 | $Q_{39}$ | $C_6H_5$ | |
| 2.40 | $Q_{40}$ | $C_6H_5$ | |
| 2.41 | $Q_{41}$ | $C_6H_5$ | |
| 2.42 | $Q_{42}$ | $C_6H_5$ | |
| 2.43 | $Q_{43}$ | $C_6H_5$ | |
| 2.44 | $Q_{44}$ | $C_6H_5$ | |
| 2.45 | $Q_{45}$ | $C_6H_5$ | |
| 2.46 | $Q_{46}$ | $C_6H_5$ | |
| 2.47 | $Q_{47}$ | $C_6H_5$ | |
| 2.48 | $Q_{48}$ | $C_6H_5$ | |
| 2.49 | $Q_{49}$ | $C_6H_5$ | |
| 2.50 | $Q_{50}$ | $C_6H_5$ | |
| 2.51 | $Q_{51}$ | $C_6H_5$ | |
| 2.52 | $Q_{52}$ | $C_6H_5$ | |
| 2.53 | $Q_{53}$ | $C_6H_5$ | |
| 2.54 | $Q_{54}$ | $C_6H_5$ | |

Tabelle 2: (Fortsetzung)

| Nr. | Q | R | Phys. Daten |
|---|---|---|---|
| 2.55 | $Q_{55}$ | $C_6H_5$ | |
| 2.56 | $Q_{56}$ | $C_6H_5$ | |
| 2.57 | $Q_{57}$ | $C_6H_5$ | |
| 2.58 | $Q_{58}$ | $C_6H_5$ | |
| 2.59 | $Q_{59}$ | $C_6H_5$ | |
| 2.60 | $Q_{60}$ | $C_6H_5$ | |
| 2.61 | $Q_{61}$ | $C_6H_5$ | |
| 2.62 | $Q_{62}$ | $C_6H_5$ | |
| 2.63 | $Q_{63}$ | $C_6H_5$ | |
| 2.64 | $Q_1$ | $CH_3$ | |
| 2.65 | $Q_2$ | $CH_3$ | |
| 2.66 | $Q_3$ | $CH_3$ | |
| 2.67 | $Q_4$ | $CH_3$ | |
| 2.68 | $Q_5$ | $CH_3$ | |
| 2.69 | $Q_6$ | $CH_3$ | |
| 2.70 | $Q_7$ | $CH_3$ | |
| 2.71 | $Q_8$ | $CH_3$ | |
| 2.72 | $Q_9$ | $CH_3$ | |
| 2.73 | $Q_{10}$ | $CH_3$ | |
| 2.74 | $Q_{11}$ | $CH_3$ | |
| 2.75 | $Q_{12}$ | $CH_3$ | |
| 2.76 | $Q_{13}$ | $CH_3$ | |
| 2.77 | $Q_{14}$ | $CH_3$ | |
| 2.78 | $Q_{15}$ | $CH_3$ | |
| 2.79 | $Q_{16}$ | $CH_3$ | |
| 2.80 | $Q_{17}$ | $CH_3$ | |
| 2.81 | $Q_{18}$ | $CH_3$ | |
| 2.82 | $Q_{19}$ | $CH_3$ | |
| 2.83 | $Q_{20}$ | $CH_3$ | |
| 2.84 | $Q_{21}$ | $CH_3$ | |
| 2.85 | $Q_{22}$ | $CH_3$ | |
| 2.86 | $Q_{23}$ | $CH_3$ | |
| 2.87 | $Q_{24}$ | $CH_3$ | |
| 2.88 | $Q_{25}$ | $CH_3$ | |
| 2.89 | $Q_{26}$ | $CH_3$ | |
| 2.90 | $Q_{27}$ | $CH_3$ | |
| 2.91 | $Q_{28}$ | $CH_3$ | |
| 2.92 | $Q_{29}$ | $CH_3$ | |
| 2.93 | $Q_{30}$ | $CH_3$ | |
| 2.95 | $Q_{32}$ | $CH_3$ | |
| 2.96 | $Q_{33}$ | $CH_3$ | |
| 2.97 | $Q_{34}$ | $CH_3$ | |
| 2.98 | $Q_{35}$ | $CH_3$ | |
| 2.99 | $Q_{36}$ | $CH_3$ | |
| 2.100 | $Q_{37}$ | $CH_3$ | |
| 2.101 | $Q_{38}$ | $CH_3$ | |
| 2.102 | $Q_{39}$ | $CH_3$ | |
| 2.103 | $Q_{40}$ | $CH_3$ | |
| 2.104 | $Q_{41}$ | $CH_3$ | |
| 2.105 | $Q_{42}$ | $CH_3$ | |
| 2.106 | $Q_{43}$ | $CH_3$ | |
| 2.107 | $Q_{44}$ | $CH_3$ | |
| 2.108 | $Q_{45}$ | $CH_3$ | |
| 2.109 | $Q_{46}$ | $CH_3$ | |
| 2.110 | $Q_{47}$ | $CH_3$ | |
| 2.111 | $Q_{48}$ | $CH_3$ | |
| 2.112 | $Q_{49}$ | $CH_3$ | |
| 2.113 | $Q_{50}$ | $CH_3$ | |
| 2.114 | $Q_{51}$ | $CH_3$ | |
| 2.115 | $Q_{52}$ | $CH_3$ | |
| 2.116 | $Q_{53}$ | $CH_3$ | |

Tabelle 2: (Fortsetzung)

| Nr. | Q | R | Phys. Daten |
|---|---|---|---|
| 2.117 | $Q_{54}$ | $CH_3$ | |
| 2.118 | $Q_{55}$ | $CH_3$ | |
| 2.119 | $Q_{56}$ | $CH_3$ | |
| 2.120 | $Q_{57}$ | $CH_3$ | |
| 2.121 | $Q_{58}$ | $CH_3$ | |
| 2.122 | $Q_{59}$ | $CH_3$ | |
| 2.123 | $Q_{60}$ | $CH_3$ | |
| 2.124 | $Q_{61}$ | $CH_3$ | |
| 2.125 | $Q_{62}$ | $CH_3$ | |
| 2.126 | $Q_{63}$ | $CH_3$ | |
| 2.127 | $Q_1$ | $C_2H_5$ | |
| 2.128 | $Q_2$ | $C_2H_5$ | |
| 2.129 | $Q_3$ | $C_2H_5$ | |
| 2.130 | $Q_4$ | $C_2H_5$ | |
| 2.131 | $Q_5$ | $C_2H_5$ | |
| 2.132 | $Q_6$ | $C_2H_5$ | |
| 2.133 | $Q_7$ | $C_2H_5$ | |
| 2.134 | $Q_8$ | $C_2H_5$ | |
| 2.135 | $Q_9$ | $C_2H_5$ | |
| 2.136 | $Q_{10}$ | $C_2H_5$ | |
| 2.137 | $Q_{11}$ | $C_2H_5$ | |
| 2.138 | $Q_{12}$ | $C_2H_5$ | |
| 2.139 | $Q_{13}$ | $C_2H_5$ | |
| 2.140 | $Q_{14}$ | $C_2H_5$ | |
| 2.141 | $Q_{15}$ | $C_2H_5$ | |
| 2.142 | $Q_{16}$ | $C_2H_5$ | |
| 2.143 | $Q_{17}$ | $C_2H_5$ | |
| 2.144 | $Q_{18}$ | $C_2H_5$ | |
| 2.145 | $Q_{19}$ | $C_2H_5$ | |
| 2.146 | $Q_{20}$ | $C_2H_5$ | |
| 2.147 | $Q_{21}$ | $C_2H_5$ | |
| 2.148 | $Q_{22}$ | $C_2H_5$ | |
| 2.149 | $Q_{23}$ | $C_2H_5$ | |
| 2.150 | $Q_{24}$ | $C_2H_5$ | |
| 2.151 | $Q_{25}$ | $C_2H_5$ | |
| 2.152 | $Q_{26}$ | $C_2H_5$ | |
| 2.153 | $Q_{27}$ | $C_2H_5$ | |
| 2.154 | $Q_{28}$ | $C_2H_5$ | |
| 2.155 | $Q_{29}$ | $C_2H_5$ | |
| 2.156 | $Q_{30}$ | $C_2H_5$ | |
| 2.158 | $Q_{32}$ | $C_2H_5$ | |
| 2.159 | $Q_{33}$ | $C_2H_5$ | |
| 2.160 | $Q_{34}$ | $C_2H_5$ | |
| 2.161 | $Q_{35}$ | $C_2H_5$ | |
| 2.162 | $Q_{36}$ | $C_2H_5$ | |
| 2.163 | $Q_{37}$ | $C_2H_5$ | |
| 2.164 | $Q_{38}$ | $C_2H_5$ | |
| 2.165 | $Q_{39}$ | $C_2H_5$ | |
| 2.166 | $Q_{40}$ | $C_2H_5$ | |
| 2.167 | $Q_{41}$ | $C_2H_5$ | |
| 2.168 | $Q_{42}$ | $C_2H_5$ | |
| 2.169 | $Q_{43}$ | $C_2H_5$ | |
| 2.170 | $Q_{44}$ | $C_2H_5$ | |
| 2.171 | $Q_{45}$ | $C_2H_5$ | |
| 2.172 | $Q_{46}$ | $C_2H_5$ | |
| 2.173 | $Q_{47}$ | $C_2H_5$ | |
| 2.174 | $Q_{48}$ | $C_2H_5$ | |
| 2.175 | $Q_{49}$ | $C_2H_5$ | |
| 2.176 | $Q_{50}$ | $C_2H_5$ | |
| 2.177 | $Q_{51}$ | $C_2H_5$ | |
| 2.178 | $Q_{52}$ | $C_2H_5$ | |

Tabelle 2: (Fortsetzung)

| Nr. | Q | R | Phys. Daten |
|---|---|---|---|
| 2.179 | $Q_{53}$ | $C_2H_5$ | |
| 2.180 | $Q_{54}$ | $C_2H_5$ | |
| 2.181 | $Q_{55}$ | $C_2H_5$ | |
| 2.182 | $Q_{59}$ | $C_2H_5$ | |

Tabelle 2: (Fortsetzung)

| Nr. | Q | R | Phys. Daten |
|---|---|---|---|
| 2.183 | $Q_{60}$ | $C_2H_5$ | |
| 2.184 | $Q_{61}$ | $C_2H_5$ | |
| 2.183 | $Q_{62}$ | $C_2H_5$ | |
| 2.184 | $Q_{63}$ | $C_2H_5$ | |

Tabelle 3:

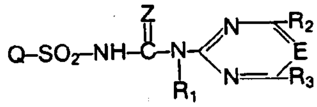

| Nr. | Q | Z | E | $R_1$ | $R_2$ | $R_3$ | Physikalische Daten |
|---|---|---|---|---|---|---|---|
| 3.1 | $Q_1$ | O | N | H | $CH_3$ | $OCH_3$ | Smp. 147 °C (Zers.) |
| 3.2 | $Q_2$ | O | N | H | $CH_3$ | $OCH_3$ | Smp. 205–206 °C |
| 3.3 | $Q_3$ | O | N | H | $CH_3$ | $OCH_3$ | |
| 3.4 | $Q_4$ | O | N | H | $CH_3$ | $OCH_3$ | |
| 3.5 | $Q_5$ | O | N | H | $CH_3$ | $OC_2H_5$ | |
| 3.6 | $Q_6$ | O | N | H | $CH_3$ | $OCH_3$ | |
| 3.7 | $Q_7$ | S | CH | H | $CH_3$ | $OC_3H_7$–n | |
| 3.8 | $Q_8$ | O | N | H | $OCH_3$ | $OCH_3$ | |
| 3.9 | $Q_9$ | O | N | H | $CH_3$ | $OCH_3$ | Smp. 152 °C (Zers.) |
| 3.10 | $Q_{10}$ | O | CH | H | $OCH_3$ | $OCH_3$ | |
| 3.11 | $Q_{11}$ | O | CH | H | $CH_3$ | $OCH_3$ | |
| 3.12 | $Q_{12}$ | O | N | H | $CH_3$ | $OCH_3$ | Smp. 60 °C |
| 3.13 | $Q_1$ | S | CH | $CH_3$ | $CH_3$ | $OCH_3$ | |
| 3.14 | $Q_1$ | S | N | $CH_3$ | $CH_3$ | $OCH_3$ | |
| 3.15 | $Q_1$ | O | N | H | $CH_3$ | $OC_3H_7$–i | |
| 3.16 | $Q_{14}$ | S | N | H | $CH_3$ | $OCH_3$ | |
| 3.17 | $Q_{15}$ | O | N | H | $OCH_3$ | $OCH_3$ | |
| 3.18 | $Q_{16}$ | O | N | H | $CH_3$ | $OCH_3$ | Smp. 160 °C (Zers.) |
| 3.19 | $Q_{17}$ | S | N | H | $SCH_3$ | $SCH_3$ | |
| 3.20 | $Q_{16}$ | O | CH | H | $OCH_3$ | $OCH_3$ | Smp. 189 °C (Zers.) |
| 3.21 | $Q_{19}$ | O | CH | H | $CH_3$ | $C_2H_5$ | |
| 3.22 | $Q_{20}$ | S | N | $CH_3$ | $CH_3$ | $CH_3$ | |
| 3.23 | $Q_{19}$ | O | N | $CH_3$ | $C_2H_5$ | $C_2H_5$ | |
| 3.24 | $Q_{22}$ | O | N | $CH_3$ | $CH_3$ | $OCH_3$ | |
| 3.25 | $Q_{13}$ | O | CH | H | $CH_3$ | $OCH_3$ | |
| 3.26 | $Q_{23}$ | O | N | H | $CH_3$ | $CH_3$ | |
| 3.27 | $Q_{24}$ | O | N | H | $CH_3$ | $OCH_3$ | |
| 3.28 | $Q_{24}$ | O | N | H | $OCH_3$ | $OCH_3$ | |
| 3.29 | $Q_{25}$ | O | N | $CH_3$ | $CH_3$ | $OCH_3$ | |
| 3.30 | $Q_{26}$ | S | N | H | $CH_3$ | $CH_3$ | |
| 3.31 | $Q_{27}$ | O | N | H | $OCH_3$ | $OCH_3$ | |
| 3.32 | $Q_{28}$ | O | N | $CH_3$ | $CH_3$ | $CH_3$ | |
| 3.33 | $Q_9$ | O | N | H | $OCH_3$ | $OCH_3$ | |
| 3.34 | $Q_{29}$ | S | CH | H | $CH_3$ | $CH_3$ | |
| 3.35 | $Q_{30}$ | O | N | H | $CH_3$ | $OCH_3$ | Smp. 171 °C (Zers.) |
| 3.37 | $O_{30}$ | O | CH | H | $CH_3$ | $OCH_3$ | Smp. Hydrochlorid 112 °C (Zers.) |
| 3.38 | $Q_{32}$ | O | CH | H | $CH_3$ | $OCH_3$ | Smp. Hydrochlorid 130 °C (Zers.) |
| 3.39 | $Q_{16}$ | O | CH | H | $CH_3$ | $OCH_3$ | Smp. Hydrochlorid 148 °C (Zers.) |
| 3.40 | $Q_{16}$ | O | CH | H | $CH_3$ | $CH_3$ | Smp. 198 °C (Zers.) |
| 3.41 | $Q_{33}$ | O | CH | H | $CH_3$ | $OCH_3$ | Smp. 214 °C (Zers.) |
| 3.42 | $Q_{34}$ | O | CH | H | $CH_3$ | $OCH_3$ | |
| 3.43 | $Q_{35}$ | O | CH | H | $CH_3$ | $OCH_3$ | Smp. 142–144 °C (Zers.) |
| 3.44 | $Q_{35}$ | O | N | H | $CH_3$ | $OCH_3$ | Smp. 143–145 °C (Zers.) |

Tabelle 3: (Fortsetzung)

| Nr. | Q | Z | E | $R_1$ | $R_2$ | $R_3$ | Physikalische Daten |
|---|---|---|---|---|---|---|---|
| 3.46 | $Q_{36}$ | O | CH | H | $CH_3$ | $OCH_3$ | Smp. 277 °C (Zers.) |
| 3.47 | $Q_{34}$ | O | N | H | $CH_3$ | $OCH_3$ | Smp. 152–153 °C (Zers.) |
| 3.48 | $Q_{36}$ | O | N | H | $CH_3$ | $OCH_3$ | Smp. 168 °C (Zers.) |
| 3.49 | $Q_{37}$ | O | CH | H | $CH_3$ | $OCH_3$ | Smp. 183 °C (Zers.) |
| 3.50 | $Q_{38}$ | O | CH | H | $CH_3$ | $OCH_3$ | Smp. 133 °C (Zers.) |
| 3.51 | $Q_{39}$ | O | CH | H | $CH_3$ | $OCH_3$ | Smp. 193 °C (Zers.) |
| 3.52 | $Q_{39}$ | O | N | H | $CH_3$ | $OCH_3$ | Smp. 187 °C (Zers.) |
| 3.53 | $Q_{18}$ | O | N | H | $CH_3$ | $OCH_3$ | |
| 3.54 | $Q_{18}$ | O | CH | H | $CH_3$ | $OCH_3$ | |
| 3.55 | $Q_{18}$ | O | CH | H | $CH_3$ | $CH_3$ | |
| 3.56 | $Q_{18}$ | O | CH | H | $CH_3$ | $OCHF_2$ | |
| 3.57 | $Q_{21}$ | O | N | H | $CH_3$ | $OCH_3$ | |
| 3.58 | $Q_{21}$ | O | CH | H | $CH_3$ | $OCH_3$ | |
| 3.59 | $Q_{21}$ | O | CH | H | $CH_3$ | $CH_3$ | |
| 3.60 | $Q_{21}$ | O | CH | H | $CH_3$ | $OCHF_2$ | |
| 3.61 | $Q_{40}$ | O | N | H | $CH_3$ | $OCH_3$ | |
| 3.62 | $Q_{41}$ | O | N | H | $CH_3$ | $OCH_3$ | |
| 3.63 | $Q_{42}$ | O | N | H | $CH_3$ | $OCH_3$ | |
| 3.64 | $Q_{43}$ | O | N | H | $CH_3$ | $OCH_3$ | |
| 3.65 | $Q_{44}$ | O | N | H | $CH_3$ | $OCH_3$ | |
| 3.66 | $Q_{45}$ | O | N | H | $CH_3$ | $OCH_3$ | |
| 3.67 | $Q_{46}$ | O | N | H | $CH_3$ | $OCH_3$ | |
| 3.68 | $Q_{47}$ | O | N | H | $CH_3$ | $OCH_3$ | |
| 3.69 | $Q_{48}$ | O | N | H | $CH_3$ | $OCH_3$ | |
| 3.70 | $Q_{49}$ | O | N | H | $CH_3$ | $OCH_3$ | |
| 3.71 | $Q_{50}$ | O | N | H | $CH_3$ | $OCH_3$ | |
| 3.72 | $Q_{51}$ | O | N | H | $CH_3$ | $OCH_3$ | |
| 3.73 | $Q_{52}$ | O | N | H | $CH_3$ | $OCH_3$ | |
| 3.74 | $Q_{53}$ | O | N | H | $CH_3$ | $OCH_3$ | |
| 3.75 | $Q_{54}$ | O | N | H | $CH_3$ | $OCH_3$ | |
| 3.76 | $Q_{55}$ | O | N | H | $CH_3$ | $OCH_3$ | |
| 3.77 | $Q_{56}$ | O | N | H | $CH_3$ | $OCH_3$ | |
| 3.78 | $Q_{57}$ | O | N | H | $CH_3$ | $OCH_3$ | |
| 3.79 | $Q_{58}$ | O | N | H | $CH_3$ | $OCH_3$ | |
| 3.80 | $Q_{59}$ | O | N | H | $CH_3$ | $OCH_3$ | |
| 3.81 | $Q_{60}$ | O | N | H | $CH_3$ | $OCH_3$ | |
| 3.82 | $Q_{61}$ | O | N | H | $CH_3$ | $OCH_3$ | |
| 3.83 | $Q_{62}$ | O | N | H | $CH_3$ | $OCH_3$ | |
| 3.84 | $Q_{63}$ | O | N | H | $CH_3$ | $OCH_3$ | |
| 3.85 | $Q_{40}$ | O | CH | H | $CH_3$ | $OCH_3$ | |
| 3.86 | $Q_{41}$ | O | CH | H | $CH_3$ | $OCH_3$ | |
| 3.87 | $Q_{42}$ | O | CH | H | $CH_3$ | $OCH_3$ | |
| 3.88 | $Q_{43}$ | O | CH | H | $CH_3$ | $OCH_3$ | |
| 3.89 | $Q_{44}$ | O | CH | H | $CH_3$ | $OCH_3$ | |
| 3.90 | $Q_{45}$ | O | CH | H | $CH_3$ | $OCH_3$ | |
| 3.91 | $Q_{46}$ | O | CH | H | $CH_3$ | $OCH_3$ | |
| 3.92 | $Q_{47}$ | Q | CH | H | $CH_3$ | $OCH_3$ | |
| 3.93 | $Q_{48}$ | O | CH | H | $CH_3$ | $OCH_3$ | |
| 3.94 | $Q_{49}$ | O | CH | H | $CH_3$ | $OCH_3$ | |
| 3.95 | $Q_{50}$ | O | CH | H | $CH_3$ | $OCH_3$ | |
| 3.96 | $Q_{51}$ | O | CH | H | $CH_3$ | $OCH_3$ | |
| 3.97 | $Q_{52}$ | Q | CH | H | $CH_3$ | $OCH_3$ | |
| 3.98 | $Q_{53}$ | O | CH | H | $CH_3$ | $OCH_3$ | |
| 3.99 | $Q_{54}$ | O | CH | H | $CH_3$ | $OCH_3$ | |
| 3.100 | $Q_{55}$ | O | CH | H | $CH_3$ | $OCH_3$ | |
| 3.101 | $Q_{56}$ | O | CH | H | $CH_3$ | $OCH_3$ | |
| 3.102 | $Q_{57}$ | O | CH | H | $CH_3$ | $OCH_3$ | |
| 3.103 | $Q_{58}$ | O | CH | H | $CH_3$ | $OCH_3$ | |
| 3.104 | $Q_{59}$ | O | CH | H | $CH_3$ | $OCH_3$ | |
| 3.105 | $Q_{60}$ | O | CH | H | $CH_3$ | $OCH_3$ | |
| 3.106 | $Q_{61}$ | O | CH | H | $CH_3$ | $OCH_3$ | |

Tabelle 3: (Fortsetzung

| Nr. | Q | Z | E | $R_1$ | $R_2$ | $R_3$ | Physikalische Daten |
|---|---|---|---|---|---|---|---|
| 3.107 | $Q_{62}$ | O | CH | H | $CH_3$ | $OCH_3$ | |
| 3.108 | $Q_{63}$ | O | CH | H | $CH_3$ | $OCH_3$ | |
| 3.109 | $Q_{40}$ | O | CH | H | $CH_3$ | $CH_3$ | |
| 3.110 | $Q_{41}$ | O | CH | H | $CH_3$ | $CH_3$ | |
| 3.111 | $Q_{42}$ | O | CH | H | $CH_3$ | $CH_3$ | |
| 3.112 | $Q_{43}$ | O | CH | H | $CH_3$ | $CH_3$ | |
| 3.113 | $Q_{44}$ | O | CH | H | $CH_3$ | $CH_3$ | |
| 3.114 | $Q_{45}$ | O | CH | H | $CH_3$ | $CH_3$ | |
| 3.115 | $Q_{46}$ | O | CH | H | $CH_3$ | $CH_3$ | |
| 3.116 | $Q_{47}$ | O | CH | H | $CH_3$ | $CH_3$ | |
| 3.117 | $Q_{48}$ | O | CH | H | $CH_3$ | $CH_3$ | |
| 3.118 | $Q_{49}$ | O | CH | H | $CH_3$ | $CH_3$ | |
| 3.119 | $Q_{50}$ | O | CH | H | $CH_3$ | $CH_3$ | |
| 3.120 | $Q_{51}$ | O | CH | H | $CH_3$ | $CH_3$ | |
| 3.121 | $Q_{52}$ | O | CH | H | $CH_3$ | $CH_3$ | |
| 3.122 | $Q_{53}$ | O | CH | H | $CH_3$ | $CH_3$ | |
| 3.123 | $Q_{54}$ | O | CH | H | $CH_3$ | $CH_3$ | |
| 3.124 | $Q_{55}$ | O | CH | H | $CH_3$ | $CH_3$ | |
| 3.125 | $Q_{56}$ | O | CH | H | $CH_3$ | $CH_3$ | |
| 3.126 | $Q_{57}$ | O | CH | H | $CH_3$ | $CH_3$ | |
| 3.127 | $Q_{58}$ | O | CH | H | $CH_3$ | $CH_3$ | |
| 3.128 | $Q_{59}$ | O | CH | H | $CH_3$ | $CH_3$ | |
| 3.129 | $Q_{60}$ | O | CH | H | $CH_3$ | $CH_3$ | |
| 3.130 | $Q_{61}$ | O | CH | H | $CH_3$ | $CH_3$ | |
| 3.131 | $Q_{62}$ | O | CH | H | $CH_3$ | $CH_3$ | |
| 3.132 | $Q_{63}$ | O | CH | H | $CH_3$ | $CH_3$ | |
| 3.133 | $Q_{40}$ | O | CH | H | $CH_3$ | $OCHF_2$ | |
| 3.134 | $Q_{41}$ | O | CH | H | $CH_3$ | $OCHF_2$ | |
| 3.135 | $Q_{42}$ | O | CH | H | $CH_3$ | $OCHF_2$ | |
| 3.136 | $Q_{43}$ | O | CH | H | $CH_3$ | $OCHF_2$ | |
| 3.137 | $Q_{44}$ | O | CH | H | $CH_3$ | $OCHF_2$ | |
| 3.138 | $Q_{45}$ | O | CH | H | $CH_3$ | $OCHF_2$ | |
| 3.139 | $Q_{46}$ | O | CH | H | $CH_3$ | $OCHF_2$ | |
| 3.140 | $Q_{47}$ | O | CH | H | $CH_3$ | $OCHF_2$ | |
| 3.141 | $Q_{48}$ | O | CH | H | $CH_3$ | $OCHF_2$ | |
| 3.142 | $Q_{49}$ | O | CH | H | $CH_3$ | $OCHF_2$ | |
| 3.143 | $Q_{50}$ | O | CH | H | $CH_3$ | $OCHF_2$ | |
| 3.144 | $Q_{51}$ | O | CH | H | $CH_3$ | $OCHF_2$ | |
| 3.145 | $Q_{52}$ | O | CH | H | $CH_3$ | $OCHF_2$ | |
| 3.146 | $Q_{53}$ | O | CH | H | $CH_3$ | $OCHF_2$ | |
| 3.147 | $Q_{54}$ | O | CH | H | $CH_3$ | $OCHF_2$ | |
| 3.148 | $Q_{55}$ | O | CH | H | $CH_3$ | $OCHF_2$ | |
| 3.149 | $Q_{56}$ | O | CH | H | $CH_3$ | $OCHF_2$ | |
| 3.150 | $Q_{57}$ | O | CH | H | $CH_3$ | $OCHF_2$ | |
| 3.151 | $Q_{58}$ | O | CH | H | $CH_3$ | $OCHF_2$ | |
| 3.152 | $Q_{59}$ | O | CH | H | $CH_3$ | $OCHF_2$ | |
| 3.153 | $Q_{60}$ | O | CH | H | $CH_3$ | $OCHF_2$ | |
| 3.154 | $Q_{61}$ | O | CH | H | $CH_3$ | $OCHF_2$ | |
| 3.155 | $Q_{62}$ | O | CH | H | $CH_3$ | $OCHF_2$ | |
| 3.156 | $Q_{63}$ | O | CH | H | $CH_3$ | $OCHF_2$ | |

Zur Applikation können die Verbindungen der Formel I in den folgenden Aufarbeitungsformen vorliegen.

Formulierungsbeispiele

Beispiel 4:

Formulierungsbeispiele für Wirkstoffe der Formel I (% = Gewichtsprozent)

| a) Spritzpulver | a) | b) | c) |
|---|---|---|---|
| Wirkstoff | 20% | 60% | 0,5% |
| Na-Ligninsulfonat | 5% | 5% | 5% |
| Na-Laurylsulfat | 3% | – | – |
| Na-Diisobutylnaphthalin-sulfonat | – | 6% | 6% |
| Octylphenolpolyäthylen-glykoläther (7–8 Mol AeO) | – | 2% | 2% |
| Hochdisperse Kieselsäure | 5% | 27% | 27% |
| Kaolin | 67% | – | – |
| Natriumchlorid | – | – | 59,5% |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsion-Konzentrat | a) | b) |
|---|---|---|
| Wirkstoff | 10% | 1% |
| Octylphenolpolyäthylenglykol-äther (4–5 Mol AeO) | 3% | 3% |
| Ca-Dodecylbenzolsulfonat | 3% | 3% |
| Ricinusölpolyglykoläther (36 Mol AeO) | 4% | 4% |
| Cyclohexanon | 30% | 10% |
| Xylolgemisch | 50% | 79% |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff | 0,1% | 1% |
| Talkum | 99,9% | – |
| Kaolin | – | 99% |

Man erhält anwendungsfertige Stäubemittel, indem der Wirkstoff mit dem Träger vermischt und auf einer geeigneten Mühle vermahlen wird.

| d) Extruder Granulat | a) | b) |
|---|---|---|
| Wirkstoff | 10% | 1% |
| Na-Ligninsulfonat | 2% | 2% |
| Carboxymethylcellulose | 1% | 1% |
| Kaolin | 87% | 96% |

Der Wirkstoff wird mit den Zusatzstoffen vermischt, vermahlen und mit Wasser angefeuchtet. Dieses Gemisch wird extrudiert und anschliessend im Luftstrom getrocknet.

| e) Umhüllungs-Granulat | |
|---|---|
| Wirkstoff | 3% |
| Polyäthylenglykol (MG 200) | 3% |
| Kaolin | 94% |

Der feingemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| f) Suspensions-Konzentrat | a) | b) |
|---|---|---|
| Wirkstoff | 40% | 5% |
| Äthylenglykol | 10% | 10% |
| Nonylphenolpolyäthylenglykol-äther (15 Mol AeO) | 6% | 1% |
| Na-Ligninsulfonat | 10% | 5% |
| Carboxymethylcellulose | 1% | 1% |
| 37%ige wässrige Formaldehyd-Lösung | 0,2% | 0,2% |
| Silikonöl in Form einer 75%igen wässrigen Emulsion | 0,8% | 0,8% |
| Wasser | 32% | 77% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

| g) Salzlösung | |
|---|---|
| Wirkstoff | 5% |
| Isopropylamin | 1% |
| Octylphenolpolyäthylenglykoläther (78 Mol AeO) | 3% |
| Wasser | 91% |

Aus solchen Konzentraten können durch Verdünnen mit Wasser Emulsionen der gewünschten Anwendungskonzentration hergestellt werden, die besonders zur Blattapplikation geeignet sind. Darüberhinaus können weitere Spritzulver mit anderen Mischungsverhältnissen oder anderen in der Formulierungstechnik gebräuchlichen Trägermaterialien und Zuschlagstoffen hergestellt werden. Die Wirkstoffe werden in geeigneten Mischern mit den genannten Zuschlagstoffen innig vermischt und auf entsprechenden Mühlen und Walzen vermahlen. Man erhält Spritzpulver von vorzüglicher Benetzbarkeit und Schwebefähigkeit, die sich mit Wasser zu Suspensionen der gewünschten Konzentration verdünnt und insbesondere zur Blattapplikation verwenden lassen. Solche Mittel gehören ebenfalls zur Erfindung.

Mittel, die nach der oben beschriebenen Art formuliert wurden und als Wirkungskomponente eine der Verbindungen aus der Tabelle 1 enthielten, liessen sich mit sehr gutem Erfolg pflanzenwuchsregulatorisch und/oder herbizid einsetzen.

Biologische Beispiele:

Beispiel 5:

Herbizidwirkung vor dem Auflaufen der Pflanzen

Kunststofftöpfe werden mit expandiertem Vermiculit (Dichte 0,135 g/cm$^3$, Wasserabsorptionsvermögen: 0,565 l/l) gefüllt. Nach dem Sättigen des nicht adsorptiven Vermiculits mit einer wässrigen Wirkstoffemulsion in deonisiertem Wasser, die die Wirkstoffe in einer Konzentration von 70,8 ppm enthält, werden Samen der

folgenden Pflanzen auf die Oberfläche gesät: Nasturtium officinalis, Agrostis tenuis, Stellaria media und Digitaria sanguinalis. Die Versuchsgefässe werden anschliessend in einer Klimakammer bei 20°C, einer Beleuchtung von ca. 20 kLux und einer relativen Luftfeuchtigkeit von 70% gehalten. Während der Keimphase von 4 bis 5 Tagen werden die Töpfe zur Erhöhung der örtlichen Luftfeuchtigkeit mit lichtdurchlässigem Material abgedeckt und mit deonisiertem Wasser gegossen. Nach dem 5. Tag wird dem Giesswasser 0,5% eines handelsüblichen Flüssigdüngers (®Greenzit) zugesetzt. 12 Tage nach der Aussaat wird der Versuch ausgewertet und die Wirkung auf die Versuchspflanzen nach dem folgenden Massstab bewertet:

1: Pflanze nicht gekeimt oder total abgestorben
2-3: sehr starke Wirkung
4-6: mittlere Wirkung
7-8: schwache Wirkung
9: keine Wirkung (wie unbehandelte Kontrolle).

Pre-emergente Wirkung:
Konzentration der Wirkstoffemulsion: 70,8 ppm

| Testpflanze Wirkstoff Nr. | Nasturtium | Stellaria | Agrostis | Digitaria |
|---|---|---|---|---|
| 3.18 | 2 | 6 | 2 | 6 |
| 3.35 | 2 | 2 | 2 | 3 |
| 3.37 | 1 | 2 | 1 | 2 |
| 3.38 | 2 | 3 | 2 | 3 |
| 3.39 | 2 | 2 | 2 | 2 |
| 3.40 | 2 | 2 | 1 | 2 |
| 3.41 | 2 | 3 | 2 | 3 |
| 3.42 | 2 | 5 | 2 | 5 |
| 3.43 | 1 | 2 | 1 | 2 |
| 3.44 | 1 | 3 | 1 | 3 |
| 3.46 | 1 | 2 | 1 | 3 |
| 3.47 | 2 | 4 | 2 | 7 |
| 3.48 | 2 | 7 | 2 | 8 |
| 3.49 | 2 | 2 | 2 | 3 |

Beispiel 6:

Nachweis der Herbizidwirkung nach dem Auflaufen der Pflanzen (Kontaktwirkung)

Eine grössere Anzahl Unkräuter und Kulturpflanzen, sowohl monokotyle wie dikotyle, wurden nach dem Auflaufen, im 4- bis 6-Blattstadium mit einer wässrigen Wirkstoffdispersion in verschiedenen Dosierungen auf die Pflanzen gespritzt und diese bei 24° bis 26°C und 45-60% relativer Luftfeuchtigkeit gehalten. 15 Tage nach der Behandlung zeigten die Verbindungen der Formel I gute Herbizidwirkung.

Beispiel 7:

Wuchshemmung bei Getreide

In Kunststomöpfen mit sterilisierter Erde wurden die Getreidearten Hordeum vulgare (Sommergerste) und Secale (Sommerroggen) im Gewächshaus angesät und nach Bedarf bewässert. Die Sprösslinge wurden ca. 21 Tage nach der Aussaat mit der wässrigen Spritzbrühe eines Wirkstoffes der Formel I besprüht. Die Wirkstoffmenge betrug umgerechnet 0,5 bzw. 2,5 kg Aktivsubstanz per Hektar. 10 und 21 Tage nach Applikation wurde das Wachstum des Getreides beurteilt. Hierbei konnte festgestellt werden, dass Getreidepflanzen die mit Wirkstoffen der Formel I behandelt worden waren, im Vergleich zu unbehandelten Kontrollpflanzen eine starke Wuchsreduktion aufwiesen.

Beispiel 8:

Wuchshemmung bei Gräsern

In Kunststoffschalen mit Erde-Topf-Sand- Gemisch (6:3:1) wurden die Gräser Lolium perenne, Poa pratensis, Festuca ovina und Cynodon dactylon im Gewächshaus angesät und nach Bedarf bewässert. Die aufgelaufenen Gräser wurden wöchentlich bis auf 4 cm Höhe zurückgeschnitten und ca. 50 Tage nach der Aus-

saat und einen Tag nach dem letzten Schnitt mit der wässrigen Spritzbrühe eines Wirkstoffes der Formel I besprüht. Die Wirkstoffmenge betrug umgerechnet 2,5 kg Aktivsubstanz per Hektar. 10 und 21 Tage nach Applikation wurde das Wachstum der Gräser beurteilt, dabei zeigte es sich, dass die erfindungsgemässen Wirkstoffe aus der Tabelle 3 eine merkliche Wuchshemmung bewirkten.

## Patentansprüche

## Patentansprüche für folgende Vertragsstaaten : CH, DE, FR, GB, IT, LI, NL

1. Verbindungen der Formel

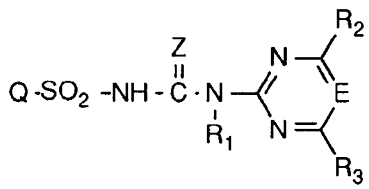

worin
$R_1$ Wasserstoff oder $C_1$-$C_5$-Alkyl,
$R_2$ und $R_3$ unabhängig voneinander Wasserstoff, $C_1$-$C_5$-Alkyl, $C_1$-$C_5$-Alkoxy, $C_1$-$C_5$-Alkylthio, $C_1$-$C_5$-Halogenalkyl, Halogen, $C_1$-$C_5$-Halogenalkoxy, $C_1$-$C_5$-Halogenalkylthio, $C_1$-$C_5$-Alkylamino, Di-$C_1$-$C_4$-alkylamino, eine Alkoxyalkylgruppe oder Alkoxyalkoxygruppe mit maximal 6 Kohlenstoffatomen bedeuten;
Z für Sauerstoff oder Schwefel steht;
E für -CH= oder -N= steht und
Q einen über ein Kohlenstoffatom gebundenen, gegebenenfalls substituierten fünfgliedrigen heterocyclischen Rest, ausgewählt aus der Reihe Pyrazol, Imidazol, 1,2,4-Triazol, 1,2,3-Triazol, Oxazol, Thiazol, Isooxazol, Isothiazol, Furazan, 1,2,4-Thiadiazol, 1,2,4-Oxadiazol, 1,3,4-Oxadiazol, 1,2,3-Thiadiazol und 1,3,4-Thiadiazol, bedeutet, wobei die Substituenten aus der Gruppe Halogen, Pseudohalogen, Nitro, Alkyl, Haloalkyl, Alkoxy, Alkylthio, Hydroxy, Haloalkoxy, Haloalkylthio, Amino, Alkylamino, Dialkylamino, Alkylcarbonylamino, Alkylcarbonyl, Alkoxycarbonyl, Methoxypropyl, Alkylthiocarbonyl, Carbamoyl, Alkylaminocarbonyl, Dialkylaminocarbonyl, Alkylsulfinyl, Alkylsulfonyl, Alkenyloxy, Alkinyloxy oder ein gegebenenfalls durch Alkylcarbonyl, Alkoxycarbonyl, Halogen, Nitro, Cyano, Alkyl, Alkoxy, Haloalkyl oder Haloalkoxy substituiertes Phenyl, Phenoxy, Phenylthio oder eine gegebenenfalls durch Halogen und/oder Alkyl substituierte Benzylgruppe gewählt sind, mit der Massgabe, dass der Rest Pyrazol-4-yl nicht mit umfasst ist;
unter Einschluss ihrer Salze.

2. Verbindungen der Formel I nach Anspruch 1, dadurch gekennzeichnet, dass Z für Sauerstoff und $R_1$ für Wasserstoff steht.

3. Verbindungen der Formel I nach Anspruch 1, dadurch gekennzeichnet, dass $R_1$ für Wasserstoff, $R_2$ und $R_3$ für $C_1$-$C_4$-Alkyl oder $C_1$-$C_4$-Alkoxy und Q und Z sowie E die unter Formel I angegebenen Bedeutungen haben.

4. Verbindungen der Formel I nach Anspruch 1, dadurch gekennzeichnet, dass $R_1$ für Wasserstoff steht, $R_2$ und $R_3$ $C_1$-$C_3$-Alkyl oder $C_1$-$C_3$-Alkoxy, Z Sauerstoff, E Stickstoff oder CH bedeuten, Q für einen unter Formel I definierten heterocyclischen Rest steht, der gegebenenfalls durch Chlor, Brom, Fluor, Nitro, Cyano, $C_1$-$C_4$-Alkylcarbonyl, $C_1$-$C_4$-Alkyloxycarbonyl, $C_1$-$C_3$-Alkoxy, $C_1$-$C_3$-Alkylthio, $C_1$-$C_3$-Alkylsulfonyl, Methoxypropyl, $C_1$-$C_3$-Alkylsulfonyl, $C_1$-$C_4$-Alkyl, Trifluormethyl, Trichlormethyl, Cyano-$C_1$-$C_4$-Alkyl, $C_2$-$C_4$-Alkenyloxy, $C_3$-$C_4$-Alkinyloxy oder N-$C_1$-$C_4$-Alkylcarbonylamino substituiert ist.

5. N-[1-(3-Methoxy-1-propyl)-imidazol-2-ylsulfonyl]-N'-(4-methoxy-6-methyl-pyrimidin-2-yl)-harnstoff gemäss Anspruch 1.

6. Verfahren zur Herstellung von Verbindungen der Formel I nach Anspruch 1, dadurch gekennzeichnet,

dass man ein Phenylsulfonylderivat der Formel II

$$Q\text{-}SO_2W \qquad (II)$$

mit einer Verbindung der Formel III

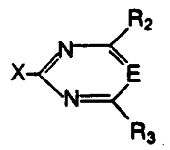

(III)

umsetzt, wobei X und W für eine der Gruppen $-NH_2$, $-N = C = Z$ oder $-NR_1-CZ-OR$ stehen; die Substituenten $R_1$, $R_2$, $R_3$, Q, Z und E die unter Formel I angegebenen Bedeutungen haben, R für einen aliphatischen oder aromatischen Reste steht; mit der Massgabe, dass die Reaktionspartner der Formeln II und III stets so gewählt werden, dass eine Aminofunktion entweder mit einer Isocyanato-, Isothiocyanato- oder mit einer $[-NR_1-CZ-OR]$-Gruppe zur Reaktion gelangt.

7. Verfahren nach Anspruch 6, dadurch gekennzeichnet, dass R für $C_1$-$C_4$-Alkyl, Phenyl oder Benzyl steht.

8. Verfahren nach Anspruch 6, dadurch gekennzeichnet, dass man in Gegenwart eines reaktionsinerten Lösungsmittels oder eines Lösungsmittelgemisches arbeitet.

9. Verfahren nach Anspruch 8, dadurch gekennzeichnet, dass die Reaktion in Gegenwart einer Base durchgeführt wird.

10. Verfahren nach Anspruch 8, dadurch gekennzeichnet, dass man die Umsetzung bei Temperaturen von -20° bis + 120 °C durchführt.

11. Herbizides und/oder pflanzenwuchsregulierendes Mittel, dadurch gekennzeichnet, dass es als mindestens eine aktive Komponente eine Verbindung der Formel I nach Anspruch 1 enthält.

12. Mittel nach Anspruch 11, dadurch gekennzeichnet, dass es 0,1 bis 95 Gewichtsprozent einer Verbindung der Formel I nach Anspruch 1 und 1 bis 99,9 Gewichtsprozent an Zuschlagstoffen, darunter 0,1 bis 25 Gewichtsprozent eines Tensides enthält.

13. Verwendung einer Verbindung der Formel I nach Anspruch 1 zur Bekämpfung von unerwünschten Pflanzensorten in Kulturen von Nutzpflanzen und/oder zur Regulierung des Pflanzenwachstums von Nutzpflanzen.

14. Verwendung nach Anspruch 13 zur selektiven Unkrautbekämpfung.

15. Verwendung nach Anspruch 13 zur Hemmung des Pflanzenwuchses.

16. Verfahren zur pre- oder post-emergenten Bekämpfung von Unkräutern und/oder zur Beeinflussung des Wachstums von Nutzpflanzen, dadurch gekennzeichnet, dass man eine wirksame Menge eines Wirkstoffes der Formel I gemäss Anspruch 1 auf die Schad- oder Nutzpflanze, deren Pflanzenteile oder deren Standort appliziert.

17. Verbindungen der Formel

$$Q\text{-}SO_2\text{-}NH\text{-}CZ\text{-}OR$$

worin Q und Z die unter Formel I im Anspruch 1 gegebene Bedeutung haben und R für einen aliphatischen oder aromatischen Rest steht.

18. Verbindungen gemäss Anspruch 17, dadurch gekennzeichnet, dass R für $C_1$-$C_4$-Alkyl, Phenyl oder Benzyl steht.

**Patentansprüche für folgenden Vertragsstaat : AT**

1. Herbizides und/oder pflanzenwuchsregulierendes Mittel, dadurch gekennzeichnet, dass es als mindestens eine aktive Komponente eine Verbindung der Formel I

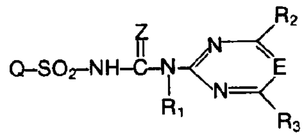

(I)

oder ein Salz davon enthält,
worin

$R_1$ Wasserstoff oder $C_1$-$C_5$-Alkyl,

$R_2$ und $R_3$ unabhängig voneinander Wasserstoff, $C_1$-$C_5$-Alkyl, $C_1$-$C_5$-Alkoxy, $C_1$-$C_5$-Alkylthio, $C_1$-$C_5$-Halogenalkyl, Halogen, $C_1$-$C_5$-Halogenalkoxy, $C_1$-$C_5$-Halogenalkylthio, $C_1$-$C_5$-Alkylamino, Di-$C_1$-$C_4$-alkylamino, eine Alkoxyalkylgruppe oder Alkoxyalkoxygruppe mit maximal 6 Kohlenstoffatomen bedeuten;

Z für Sauerstoff oder Schwefel steht;

E für -CH= oder -N= steht und

Q einen über ein Kohlenstoffatom gebundenen, gegebenenfalls substituierten fünfgliedrigen heterocyclischen Rest, ausgewählt aus der Reihe Pyrazol, Imidazol, 1,2,4-Triazol, 1,2,3-Triazol, Oxazol, Thiazol, Isooxazol, Isothiazol, Furazan, 1,2,4-Thiadiazol, 1,2,4-Oxadiazol, 1,3,4-Oxadiazol, 1,2,3-Thiadiazol und 1,3,4-Thiadiazol bedeutet, wobei die Substituenten aus der Gruppe Halogen, Pseudohalogen, Nitro, Alkyl, Haloalkyl, Alkoxy, Alkylthio, Hydroxy, Haloalkoxy, Haloalkylthio, Amino, Alkylamino, Dialkylamino, Alkylcarbonylamino, Alkylcarbonyl, Alkoxycarbonyl, Methoxypropyl, Alkylthiocarbonyl, Carbamoyl, Alkylaminocarbonyl, Dialkylaminocarbonyl, Alkylsulfinyl, Alkylsulfonyl, Alkenyloxy, Alkinyloxy oder ein gegebenenfalls durch Alkylcarbonyl, Alkoxycarbonyl, Halogen, Nitro, Cyano, Alkyl, Alkoxy, Haloalkyl oder Haloalkoxy substituiertes Phenyl, Phenoxy, Phenylthio oder eine gegebenenfalls durch Halogen und/oder Alkyl substituierte Benzylgruppe gewählt sind.

2. Mittel gemäss Anspruch 1, dadurch gekennzeichnet, dass Z für Sauerstoff und $R_1$ für Wasserstoff steht.

3. Mittel gemäss Anspruch 1, dadurch gekennzeichnet, dass $R_1$ für Wasserstoff, $R_2$ und $R_3$ für $C_1$-$C_4$-Alkyl oder $C_1$-$C_4$-Alkoxy und Q und Z sowie E die unter Formel I angegebenen Bedeutungen haben.

4. Mittel gemäss Anspruch 1, dadurch gekennzeichnet, dass $R_1$ für Wasserstoff steht, $R_2$ und $R_3$ $C_1$-$C_3$-Alkyl oder $C_1$-$C_3$-Alkoxy, Z Sauerstoff, E Stickstoff oder CH bedeuten, Q für einen unter Formel I definierten heterocyclischen Rest steht, der gegebenenfalls durch Chlor, Brom, Fluor, Nitro, Cyano, $C_1$-$C_4$-Alkylcarbonyl, $C_1$-$C_4$-Alkyloxycarbonyl, $C_1$-$C_3$-Alkoxy, $C_1$-$C_3$-Alkylthio, $C_1$-$C_3$-Alkylsulfonyl, Methoxypropyl, $C_1$-$C_3$-Alkylsulfonyl, $C_1$-$C_4$-Alkyl, Trifluormethyl, Trichlormethyl, Cyano-$C_1$-$C_4$-Alkyl, $C_2$-$C_4$-Alkenyloxy, $C_3$-$C_4$-Alkinyloxy oder N-$C_1$-$C_4$-Alkylcarbonylamino substituiert ist.

5. Mittel gemäss Anspruch 1, dadurch gekennzeichnet, dass es als Wirkstoff N-[1-(3-Methoxy-1-propyl)-imidazol-2-ylsulfonyl]-N′-(4-methoxy-6-methyl-pyrimidin-2-yl)-harnstoff enthält.

6. Verfahren zur Herstellung von Verbindungen der Formel I nach Anspruch 1, dadurch gekennzeichnet, dass man ein Phenylsulfonylderivat der Formel II

$$Q\text{-}SO_2W \qquad (II)$$

mit einer Verbindung der Formel III

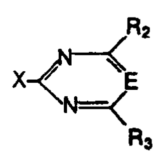

(III)

umsetzt, wobei X und W für eine der Gruppen $-NH_2$, -N = C = Z oder $-NR_1$-CZ-OR stehen; die Substituenten $R_1$, $R_2$, $R_3$, Q, Z und E die unter Formel I angegebenen Bedeutungen haben, R für einen aliphatischen oder aromatischen Reste steht; mit der Massgabe, dass die Reaktionspartner der Formeln II und III stets so gewählt werden, dass eine Aminofunktion entweder mit einer Isocyanato-, Isothiocyanato- oder mit einer [$-NR_1$-CZ-OR]-Gruppe zur Reaktion gelangt.

7. Verfahren nach Anspruch 6, dadurch gekennzeichnet, dass R für $C_1$-$C_4$-Alkyl, Phenyl oder Benzyl steht.

8. Verfahren nach Anspruch 6, dadurch gekennzeichnet, dass man in Gegenwart eines reaktionsinerten Lösungsmittels oder eines Lösungsmittelgemisches arbeitet.

9. Verfahren nach Anspruch 8, dadurch gekennzeichnet, dass die Reaktion in Gegenwart einer Base durchgeführt wird.

10. Verfahren nach Anspruch 8, dadurch gekennzeichnet, dass man die Umsetzung bei Temperaturen von -20° bis + 120 °C durchführt.

11. Mittel nach Anspruch 1, dadurch gekennzeichnet, dass es 0,1 bis 95 Gewichtsprozent einer Verbindung der Formel I nach Anspruch 1 und 1 bis 99,9 Gewichtsprozent an Zuschlagstoffen, darunter 0,1 bis 25 Gewichtsprozent eines Tensides enthält.

12. Verwendung einer Verbindung der Formel I nach Anspruch 1 zur Bekämpfung von unerwünschten Pflanzensorten in Kulturen von Nutzpflanzen und/oder zur Regulierung des Pflanzenwachstums von Nutzpflanzen.

13. Verwendung nach Anspruch 12 zur selektiven Unkrautbekämpfung.

14. Verwendung nach Anspruch 12 zur Hemmung des Pflanzenwuchses.

15. Verfahren zur pre- oder post-emergenten Bekämpfung von Unkräutern und/oder zur Beeinflussung des Wachstums von Nutzpflanzen, dadurch gekennzeichnet, dass man eine wirksame Menge eines Wirkstoffes der Formel I gemäss Anspruch 1 auf die Schad- oder Nutzpflanze, deren Pflanzenteile oder deren Standort appliziert.

**Patentansprüche für folgenden Vertragsstaat : BE**

1. Verbindungen der Formel I

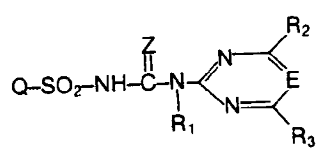

(I)

worin

$R_1$ Wasserstoff oder $C_1$-$C_5$-Alkyl,

$R_2$ und $R_3$ unabhängig voneinander Wasserstoff, $C_1$-$C_5$-Alkyl, $C_1$-$C_5$-Alkoxy, $C_1$-$C_5$-Alkylthio, $C_1$-$C_5$-Halogenalkyl, Halogen, $C_1$-$C_5$-Halogenalkoxy, $C_1$-$C_5$-Halogenalkylthio, $C_1$-$C_5$-Alkylamino, Di-$C_1$-$C_4$-alkylamino, eine Alkoxyalkylgruppe oder Alkoxyalkoxygruppe mit maximal 6 Kohlenstoffatomen bedeuten;

Z für Sauerstoff oder Schwefel steht;

E für -CH= oder -N= steht und

Q einen über ein Kohlenstoffatom gebundenen, gegebenenfalls substituierten fünfgliedrigen heterocyclischen Rest, ausgewählt aus der Reihe Pyrazol, Imidazol, 1,2,4-Triazol, 1,2,3-Triazol, Oxazol, Thiazol, Isooxazol, Isothiazol, Furazan, 1,2,4-Thiadiazol, 1,2,4-Oxadiazol, 1,3,4-Oxadiazol, 1,2,3-Thiadiazol und 1,3,4-Thiadiazol, bedeutet, wobei die Substituenten aus der Gruppe Halogen, Pseudohalogen, Nitro, Alkyl, Haloalkyl, Alkoxy, Alkylthio, Hydroxy, Haloalkoxy, Haloalkylthio, Amino, Alkylamino, Dialkylamino, Alkylcarbonylamino, Alkylcarbonyl, Alkoxycarbonyl, Methoxypropyl, Alkylthiocarbonyl, Carbamoyl, Alkylaminocarbonyl, Dialkylaminocarbonyl, Alkylsulfinyl, Alkylsulfonyl, Alkenyloxy, Alkinyloxy oder ein gegebenenfalls durch Alkylcarbonyl, Alkoxycarbonyl, Halogen, Nitro, Cyano, Alkyl, Alkoxy, Haloalkyl oder Haloalkoxy substituiertes Phenyl, Phenoxy, Phenylthio oder eine gegebenenfalls durch Halogen und/oder Alkyl substituierte Benzylgruppe gewählt sind;
unter Einschluss ihrer Salze.

2. Verbindungen der Formel I nach Anspruch 1, dadurch gekennzeichnet, dass Z für Sauerstoff und $R_1$ für Wasserstoff steht.

3. Verbindungen der Formel I nach Anspruch 1, dadurch gekennzeichnet, dass $R_1$ für Wasserstoff, $R_2$ und $R_3$ für $C_1$-$C_4$-Alkyl oder $C_1$-$C_4$-Alkoxy und Q und Z sowie E die unter Formel I angegebenen Bedeutungen haben.

4. Verbindungen der Formel I nach Anspruch 1, dadurch gekennzeichnet, dass $R_1$ für Wasserstoff steht, $R_2$ und $R_3$ $C_1$-$C_3$-Alkyl oder $C_1$-$C_3$-Alkoxy, Z Sauerstoff, E Stickstoff oder CH bedeuten, Q für einen unter Formel I definierten heterocyclischen Rest steht, der gegebenenfalls durch Chlor, Brom, Fluor, Nitro, Cyano, $C_1$-$C_4$-Alkylcarbonyl, $C_1$-$C_4$-Alkyloxycarbonyl, $C_1$-$C_3$-Alkoxy, $C_1$-$C_3$-Alkylthio, $C_1$-$C_3$-Alkylsulfonyl, Methoxypropyl, $C_1$-$C_3$-Alkylsulfonyl, $C_1$-$C_4$-Alkyl, Trifluormethyl, Trichlormethyl, Cyano-$C_1$-$C_4$-Alkyl, $C_2$-$C_4$-Alkenyloxy, $C_3$-$C_4$-Alkinyloxy oder N-$C_1$-$C_4$-Alkylcarbonylamino substituiert ist.

5. N-[1-(3-Methoxy-1-propyl)-imidazol-2-ylsulfonyl]-N'-(4-methoxy-6-methyl-pyrimidin-2-yl)-harnstoff gemäss Anspruch 1.

6. Verfahren zur Herstellung von Verbindungen der Formel I nach Anspruch 1, dadurch gekennzeichnet, dass man ein Phenylsulfonylderivat der Formel II

$$Q\text{-}SO_2W \qquad (II)$$

mit einer Verbindung der Formel III

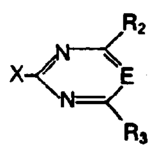

(III)

umsetzt, wobei X und W für eine der Gruppen $-NH_2$, $-N = C = Z$ oder $-NR_1$-CZ-OR stehen; die Substituenten $R_1$, $R_2$, $R_3$, Q, Z und E die unter Formel I angegebenen Bedeutungen haben, R für einen aliphatischen oder aromatischen Reste steht; mit der Massgabe, dass die Reaktionspartner der Formeln II und III stets so gewählt werden, dass eine Aminofunktion entweder mit einer Isocyanato-, Isothiocyanato- oder mit einer [$-NR_1$-CZ-OR]-Gruppe zur Reaktion gelangt.

7. Verfahren nach Anspruch 6, dadurch gekennzeichnet, dass R für $C_1$-$C_4$-Alkyl, Phenyl oder Benzyl steht.

8. Verfahren nach Anspruch 6, dadurch gekennzeichnet, dass man in Gegenwart eines reaktionsinerten Lösungsmittels oder eines Lösungsmittelgemisches arbeitet.

9. Verfahren nach Anspruch 8, dadurch gekennzeichnet, dass die Reaktion in Gegenwart einer Base durchgeführt wird.

10. Verfahren nach Anspruch 8, dadurch gekennzeichnet, dass man die Umsetzung bei Temperaturen von -20° bis + 120 °C durchführt.

11. Herbizides und/oder pflanzenwuchsregulierendes Mittel, dadurch gekennzeichnet, dass es als mindestens eine aktive Komponente eine Verbindung der Formel I nach Anspruch 1 enthält.

12. Mittel nach Anspruch 11, dadurch gekennzeichnet, dass es 0,1 bis 95 Gewichtsprozent einer Verbindung der Formel I nach Anspruch 1 und 1 bis 99,9 Gewichtsprozent an Zuschlagstoffen, darunter 0,1 bis 25 Gewichtsprozent eines Tensides enthält.

13. Verwendung einer Verbindung der Formel I nach Anspruch 1 zur Bekämpfung von unerwünschten Pflanzensorten in Kulturen von Nutzpflanzen und/oder zur Regulierung des Pflanzenwachstums von Nutzpflanzen.

14. Verwendung nach Anspruch 13 zur selektiven Unkrautbekämpfung.

15. Verwendung nach Anspruch 13 zur Hemmung des Pflanzenwuchses.

16. Verfahren zur pre- oder post-emergenten Bekämpfung von Unkräutern und/oder zur Beeinflussung des Wachstums von Nutzpflanzen, dadurch gekennzeichnet, dass man eine wirksame Menge eines Wirkstoffes der Formel I gemäss Anspruch 1 auf die Schad- oder Nutzpflanze, deren Pflanzenteile oder deren Standort appliziert.

17. Verbindungen der Formel

$$Q\text{-}SO_2\text{-}NH\text{-}CZ\text{-}OR$$

worin Q und Z die unter Formel I im Anspruch 1 gegebene Bedeutung haben und R für einen aliphatischen oder aromatischen Rest steht.

18. Verbindungen gemäss Anspruch 17, dadurch gekennzeichnet, dass R für $C_1$-$C_4$-Alkyl, Phenyl oder Benzyl steht.

## Claims

### Claims for the following Contracting States : CH, DE, FR, GB, IT, LI, NL

1. Compounds of formula I

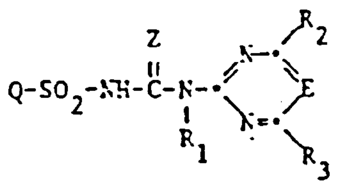

(I)

wherein

| | |
|---|---|
| $R_1$ | is hydrogen or $C_1$-$C_5$alkyl, |
| $R_2$ and $R_3$ | independently of one another are each hydrogen, $C_1$-$C_5$alkyl, $C_1$-$C_5$alkoxy, $C_1$-$C_5$alkylthio, $C_1$-$C_5$haloalkyl, halogen, $C_1$-$C_5$haloalkoxy, $C_1$-$C_5$haloalkylthio, $C_1$-$C_5$alkylamino, di-$C_1$-$C_4$alkylamino, or an alkoxyalkyl or alkoxyalkoxy group each having a maximum of 6 carbon atoms, |
| Z | is oxygen or sulphur, |
| E | is -CH= or -N=, and |
| Q | is a five-membered heterocyclic radical selected from the group consisting of pyrazole, imidazole, 1,2,4-triazole, 1,2,3-triazole, oxazole, thiazole, isooxazole, isothiazole, furazan, 1,2,4-thiadiazole, 1,2,4-oxadiazole, 1,3,4-oxadiazole, 1,2,3-thiadiazole and 1,3,4-thiadiazole, which is bonded by way of a carbon atom and is unsubstituted or substituted by halogen, pseudohalogen, nitro, alkyl, haloalkyl, alkoxy, alkylthio, hydroxy, haloalkoxy, haloalkylthio, amino, alkylamino, dialkylamino, alkylcarbonylamino, alkylcarbonyl, alkoxycarbonyl, methoxypropyl, alkylthiocarbonyl, carbamoyl, alkylaminocarbonyl, dialkylaminocarbonyl, alkylsulphinyl, alkylsulphonyl, alkenyloxy or alkynyloxy; or by a phenyl, phenoxy or phenylthio group that is unsubstituted or is substituted by alkylcarbonyl, alkoxycarbonyl, halogen, nitro, cyano, alkyl, alkoxy, haloalkyl or haloalkoxy; or by a benzyl group that is unsubstituted or is substituted by halogen and/or alkyl; with the proviso that the radical pyrazol-4-yl is not included; including the salts thereof. |

2. Compounds of the formula I according to claim 1, characterised in that Z is oxygen, and $R_1$ is hydrogen.

3. Compounds of the formula I according to claim 1, characterised in that $R_1$ is hydrogen, $R_2$ and $R_3$ are each $C_1$-$C_4$alkyl or $C_1$-$C_4$alkoxy, and Q and Z and also E are as defined under formula I.

4. Compounds of the formula I according to claim 1, characterised in that $R_1$ is hydrogen, $R_2$ and $R_3$ are each $C_1$-$C_3$alkyl or $C_1$-$C_3$alkoxy, Z is oxygen, E is nitrogen or CH, and Q is a heterocyclic radical defined under formula I, which is unsubstituted or is substituted by chlorine, bromine, fluorine, nitro, cyano, $C_1$-$C_4$alkylcarbonyl, $C_1$-$C_4$alkoxycarbonyl, $C_1$-$C_3$-alkoxy, $C_1$-$C_3$alkylthio, $C_1$-$C_3$alkylsulphonyl, methoxypropyl, $C_1$-$C_3$alkylsulphonyl, $C_1$-$C_4$alkyl, trifluoromethyl, trichloromethyl, cyano-$C_1$-$C_4$alkyl, $C_2$-$C_4$alkenyloxy, $C_3$-$C_4$-alkynyloxy or by N-$C_1$-$C_4$alkylcarbonylamino.

5. N-[1-(3-methoxy-1-propyl)imidazol-2-ylsulphonyl]-N'-(4-methoxy-6-methyl-pyrimidin-2-yl) urea according to claim 1.

6. A process for the preparation of compounds of the formula I according to claim 1, characterised in that a phenylsulphonyl derivative of the formula II

$$Q - SO_2W \qquad (II)$$

is reacted with a compound of the formula III

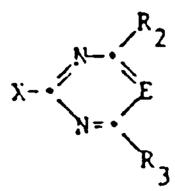

(III)

wherein X and W are each any one of the groups: $-NH_2$, $-N=C=Z$ or $-NR_1-CZ-OR$; the substituents $R_1$, $R_2$, $R_3$, Q, Z and E are as defined under formula I, R is an aliphatic or aromatic radical; with the proviso that the reactants of the formulae II and III are in all cases so selected that an amino group is reacted either with an isocyanato or isothiocyanato group or with an $[-NR_1-CZ-OR]$ group.

7. A process according to claim 6, characterised in that R is $C_1$-$C_4$alkyl, phenyl or benzyl.

8. A process according to claim 6, characterised in that the reaction is performed in the presence of a solvent or a solvent mixture inert to the reactants.

9. A process according to claim 8, characterised in that the reaction is performed in the presence of a base.

10. A process according to claim 8, characterised in that the reaction is performed at a temperature of from -20° to +120°C.

11. A herbicidal and/or plant growth-regulating composition, characterised in that it contains, as at least one active ingredient, a compound of the formula I according to claim 1.

12. A composition according to claim 11, characterised in that it contains 0.1 to 95 per cent by weight of a compound of the formula I according to claim 1, and 1 to 99.9 per cent by weight of additives, including among these 0.1 to 25 per cent by weight of a surfactant.

13. The use of a compound of the formula I according to claim 1 for controlling undesirable plant species in crops of useful plants and/or for regulating the growth of useful plants.

14. Use according to claim 13 for selectively controlling weeds.

15. Use according to claim 13 for inhibiting plant growth.

16. A method for the pre- or post-emergence controlling of weeds and/or for the influencing of the growth of useful plants, characterised in that an effective amount of an active substance of the formula I according to claim 1 is applied to the harmful plant or to the useful plant, to parts of these plants or to the locus thereof.

17. Compounds of the formula

$$Q-SO_2-NH-CZ-OR$$

wherein Q and Z are as defined under formula I in claim 1, and R is an aliphatic or aromatic radical.

18. Compounds according to claim 17, characterised in that R is $C_1$-$C_4$alkyl, phenyl or benzyl.

**Claims for the following Contracting State : AT**

1. A herbicidal and/or plant growth-regulating composition, characterised in that it contains, as at least one active ingredient, a compound of formula I

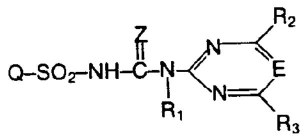

(I)

or a salt thereof,
wherein

$R_1$ is hydrogen or $C_1$-$C_5$-alkyl,

$R_2$ and $R_3$ independently of one another are each hydrogen, $C_1$-$C_5$-alkyl, $C_1$-$C_5$-alkoxy, $C_1$-$C_5$-alkylthio, $C_1$-$C_5$-haloalkyl, halogen, $C_1$-$C_5$-haloalkoxy, $C_1$-$C_5$-haloalkylthio, $C_1$-$C_5$-alkylamino, di-$C_1$-$C_4$-alkylamino, or an alkoxyalkyl or alkoxyalkoxy group each having a maximum of 6 carbon atoms,

Z is oxygen or sulphur,

E is -CH= or -N=, and

Q is a five-membered heterocyclic radical selected from the group consisting of pyrazole, imidazole, 1,2,4-triazole, 1,2,3-triazole, oxazole, thiazole, isooxazole, isothiazole, furazan, 1,2,4-thiadiazole, 1,2,4-oxadiazole, 1,3,4-oxadiazole, 1,2,3-thiadiazole and 1,3,4-thiadiazole, which is bonded by way of a carbon atom and is unsubstituted or is substituted by halogen, pseudohalogen, nitro, alkyl, haloalkyl, alkoxy, alkylthio, hydroxy, haloalkoxy, haloalkylthio, amino, alkylamino, dialkylamino, alkylcarbonylamino, alkylcarbonyl, alkoxycarbonyl, methoxypropyl, alkylthiocarbonyl, carbamoyl, alkylaminocarbonyl, dialkylaminocarbonyl, alkylsulphinyl, alkylsulphonyl, alkenyloxy or alkynyloxy; or by a phenyl, phenoxy or phenylthio group that is unsubstituted or is substituted by alkylcarbonyl, alkoxycarbonyl, halogen, nitro, cyano, alkyl, alkoxy, haloalkyl or haloalkoxy; or by a benzyl group that is unsubstituted or is substituted by halogen and/or alkyl.

2. A composition according to claim 1, characterised in that Z is oxygen, and $R_1$ is hydrogen.

3. A composition according to claim 1, characterised in that $R_1$ is hydrogen, $R_2$ and $R_3$ are each $C_1$-$C_4$-alkyl or $C_1$-$C_4$-alkoxy, and Q and Z and also E are as defined under formula I.

4. A composition according to claim 1, characterised in that $R_1$ is hydrogen, $R_2$ and $R_3$ are each $C_1$-$C_3$-alkyl or $C_1$-$C_3$-alkoxy, Z is oxygen, E is nitrogen or CH, and Q is a heterocyclic radical defined under formula I, which is unsubstituted or is substituted by chlorine, bromine, fluorine, nitro, cyano, $C_1$-$C_4$-alkylcarbonyl, $C_1$-$C_4$-alkoxycarbonyl, $C_1$-$C_3$-alkoxy, $C_1$-$C_3$-alkylthio, $C_1$-$C_3$-alkylsulphonyl, methoxypropyl, $C_1$-$C_3$-alkylsulphonyl, $C_1$-$C_4$-alkyl, trifluoromethyl, trichloromethyl, cyano-$C_1$-$C_4$-alkyl, $C_2$-$C_4$-alkenyloxy, $C_3$-$C_4$-alkynyloxy or by N-$C_1$-$C_4$-alkylcarbonylamino.

5. A composition according to claim 1, characterised in that it contains, as active ingredient, N-[1-(3-methoxy-1-propyl)-imidazol-2-ylsulphonyl]-N′-(4-methoxy-6-methyl-pyrimidin-2-yl) urea according to claim 1.

6. A process for the preparation of compounds of the formula I according to claim 1, characterised in that a phenylsulphonyl derivative of the formula II

$$Q\text{-}SO_2W \qquad (II)$$

is reacted with a compound of the formula III

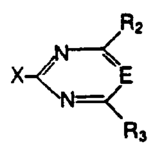

(III)

wherein X and W are each any one of the groups: $-NH_2$, -N = C = Z or $-NR_1$-CZ-OR; the substituents $R_1$, $R_2$, $R_3$, Q, Z and E are as defined under formula I, R is an aliphatic or aromatic radical; with the proviso that the reactants of the formulae II and III are in all cases so selected that an amino group is reacted either with an isocyanato or isothiocyanato group or with an [$-NR_1$-CZ-OR] group.

7. A process according to claim 6, characterised in that R is $C_1$-$C_4$-alkyl, phenyl or benzyl.

8. A process according to claim 6, characterised in that the reaction is performed in the presence of a solvent or a solvent mixture inert to the reactants.

9. A process according to claim 8, characterised in that the reaction is performed in the presence of a base.

10. A process according to claim 8, characterised in that the reaction is performed at a temperature of from - 20° to + 120 °C.

11. A composition according to claim 1, characterised in that it contains 0.1 to 95 per cent by weight of a compound of the formula I according to claim 1, and 1 to 99.9 per cent by weight of additives, including among these 0.1 to 25 per cent by weight of a surfactant.

12. The use of a compound of the formula I according to claim 1 for controlling undesirable plant species in crops of useful plants and/or for regulating the growth of useful plants.

13. Use according to claim 12 for selectively controlling weeds.

14. Use according to claim 12 for inhibiting plant growth.

15. A method fo the pre- or post-emergence controlling of weeds and/or for the influencing of the growth of useful plants, characterised in that an effective amount of an active substance of the formula I according to claim 1 is applied to the harmful plant or to the useful plant, to parts of these plants or to the locus thereof.

**Claims for the following Contracting State : BE**

1. Compounds of formula I

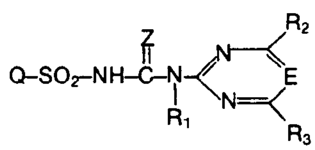

(I)

wherein

$R_1$ is hydrogen or $C_1$-$C_5$-alkyl,

$R_2$ and $R_3$ independently of one another are each hydrogen, $C_1$-$C_5$-alkyl, $C_1$-$C_5$-alkoxy, $C_1$-$C_5$-alkylthio, $C_1$-$C_5$-haloalkyl, halogen, $C_1$-$C_5$-haloalkoxy, $C_1$-$C_5$-haloalkylthio, $C_1$-$C_5$-alkylamino, di-$C_1$-$C_4$-alkylamino, or an alkoxyalkyl or alkoxyalkoxy group each having a maximum of 6 carbon atoms,

Z is oxygen or sulphur,

E is -CH= or -N=, and

Q is a five-membered heterocyclic radical selected from the group consisting of pyrazole, imidazole, 1,2,4-triazole, 1,2,3-triazole, oxazole, thiazole, isooxazole, isothiazole, furazan, 1,2,4-thiadiazole, 1,2,4-oxadiazole, 1,3,4-oxadiazole, 1,2,3-thiadiazole and 1,3,4-thiadiazole, which is bonded by way of a carbon atom and ist unsubstituted or is substituted by halogen, pseudohalogen, nitro, alkyl, haloalkyl, alkoxy, alkylthio, hydroxy, haloalkoxy, haloalkylthio, amino, alkylamino, dialkylamino, alkylcarbonylamino, alkylcarbonyl, alkoxycarbonyl, methoxypropyl, alkylthiocarbonyl, carbamoyl, alkylaminocarbonyl, dialkylaminocarbonyl, alkylsulphinyl, alkylsulphonyl, alkenyloxy or alkynyloxy; or by a phenyl, phenoxy or phenylthio group that is unsubstituted or is substituted by alkylcarbonyl, alkoxycarbonyl, halogen, nitro, cyano, alkyl, alkoxy, haloalkyl or haloalkoxy; or by a benzyl group that is unsubstituted or is substituted by halogen and/or alkyl;
including the salts thereof.

2. Compounds of the formula I according to claim 1, characterised in that Z is oxygen, and $R_1$ is hydrogen.

3. Compounds of the formula I according to claim 1, characterised in that $R_1$ is hydrogen, $R_2$ and $R_3$ are each $C_1$-$C_4$-alkyl or $C_1$-$C_4$-alkoxy, and Q and Z and also E are as defined under formula I.

4. Compounds of the formula I according to claim 1, characterised in that $R_1$ is hydrogen, $R_2$ and $R_3$ are

each $C_1$-$C_3$-alkyl or $C_1$-$C_3$-alkoxy, Z is oxygen, E is nitrogen or CH, and Q is a heterocyclic radical defined under formula I, which is unsubstituted or is substituted by chlorine, bromine, fluorine, nitro, cyano, $C_1$-$C_4$-alkylcarbonyl, $C_1$-$C_4$-alkoxycarbonyl, $C_1$-$C_3$-alkoxy, $C_1$-$C_3$-alkylthio, $C_1$-$C_3$-alkylsulphonyl, methoxypropyl, $C_1$-$C_3$-alkylsulphonyl, $C_1$-$C_4$-alkyl, trifluoromethyl, trichloromethyl, cyano-$C_1$-$C_4$-alkyl, $C_2$-$C_4$-alkenyloxy, $C_3$-$C_4$-alkynyloxy or by N-$C_1$-$C_4$-alkylcarbonylamino.

5. N-[1-(3-methoxy-1-propyl)-imidazol-2-ylsulphonyl]-N'-(4-methoxy-6-methyl-pyrimidin-2-yl) urea according to claim 1.

6. A process for the preparation of compounds of the formula I according to claim 1, characterised in that a phenylsulphonyl derivative of the formula II

$$Q\text{-}SO_2W \qquad (II)$$

is reacted with a compound of the formula III

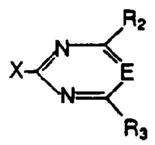

(III)

wherein X and W are each any one of the groups: $-NH_2$, $-N = C = Z$ or $-NR_1$-CZ-OR; the substituents $R_1$, $R_2$, $R_3$, Q, Z and E are as defined under formula I, R is an aliphatic or aromatic radical; with the proviso that the reactants of the formulae II and III are in all cases so selected that an amino group is reacted either with an isocyanato or isothiocyanato group or with an [$-NR_1$-CZ-OR] group.

7. A process according to claim 6, characterised in that R is $C_1$-$C_4$-alkyl, phenyl or benzyl.

8. A process according to claim 6, characterised in that the reaction is performed in the presence of a solvent or a solvent mixture inert to the reactants.

9. A process according to claim 8, characterised in that the reaction is performed in the presence of a base.

10. A process according to claim 8, characterised in that the reaction is performed at a temperature of from - 20° to + 120 °C.

11. A herbicidal and/or plant growth-regulating composition, characterised in that it contains, as at least one active ingredient, a compound of the formula I according to claim 1.

12. A composition according to claim 11, characterised in that it contains 0.1 to 95 per cent by weight of a compound of the formula I according to claim 1, and 1 to 99.9 per cent by weight of additives, including among these 0.1 to 25 per cent by weight of a surfactant.

13. The use of a compound of the formula I according to claim 1 for controlling undesirable plant species in crops of useful plants and/or for regulating the growth of useful plants.

14. Use according to claim 13 for selectively controlling weeds.

15. Use according to claim 13 for inhibiting plant growth.

16. A method for the pre- or post-emergence controlling of weeds and/or for the influencing of the growth of useful plants, characterised in that an effective amount of an active substance of the formula I according to claim 1 is applied to the harmful plant or to the useful plant, to parts of these plants or to the locus thereof.

17. Compounds of the formula

$$Q\text{-}SO_2\text{-}NH\text{-}CZ\text{-}OR$$

wherein Q and Z are as defined under formula I in claim 1, and R is an aliphatic or aromatic radical.

18. Compounds according to claim 17, characterised in that R is $C_1$-$C_4$-alkyl, phenyl or benzyl.

## Revendications

### Revendications pour les Etats contractants suivants : CH, DE, FR, GB, IT, LI, NL

1. Composés de formule I

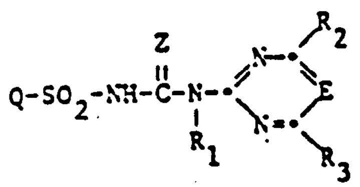

(I)

dans laquelle

| | |
|---|---|
| $R_1$ | représente l'hydrogène ou un groupe alkyle en C1-C5, |
| $R_2$ et $R_3$ | représentent chacun, indépendamment l'un de l'autre, l'hydrogène, un groupe alkyle en C1-C5, alcoxy en C1-C5, alkylthio en C1-C5, halogénoalkyle en C1-C5, un halogène, un groupe halogénoalcoxy en C1-C5, halogénoalkylthio en C1-C5, alkylamino en C1-C5, di-(alkyle en C1-C4)-amino, un groupe alcoxyalkyle ou alcoxyalcoxy contenant au maximum 6 atomes de carbone; |
| Z | représente l'oxygène ou le soufre; |
| E | représente -CH= ou -N=, et |
| Q | représente un groupe hétérocyclique à 5 chaînons éventuellement substitué, relié par l'intermédiaire d'un atome de carbone, choisi dans la classe des groupes pyrazole de l'imidazole, du 1,2,4-triazole, du 1,2,3-triazole, de l'oxazole, du thiazole, de l'isoxazole, de l'isothiazole, du furazanne, du 1,2,4-thiadiazole, du 1,2,4-oxadiazole, du 1,3,4-oxadiazole, du 1,2,3-thiadiazole et du 1,3,4-thiadiazole, les substituants étant choisis parmi les halogènes, les pseudo-halogènes, les groupes nitro, alkyle, halogénoalkyle, alcoxy, alkylthio, hydroxy, halogénoalcoxy, halogénoalkylthio, amino, alkylamino, dialkylamino, alkylcarbonylamino, alkylcarbonyle, alcoxycarbonyle, méthoxypropyle, alkylthiocarbonyle, carbamoyle, alkylaminocarbonyle, dialkylaminocarbonyle, alkylsulfinyle, alkylsulfonyle, alcényloxy, alcynyloxy ou un groupe phényle,phénoxy, phénylthio éventuellement substitué par des groupes alkylcarbonyle, alcoxycarbonyle, des halogènes, des groupes nitro, cyano, alkyle, alcoxy, halogénoalkyle ou halogénoalcoxy, ou un groupe benzyle éventuellement substitué par des halogènes et/ou des groupes alkyle; à l'exclusion du groupe Pyrazol-4-yl; |

y compris leurs sels.

2. Composés de formule I selon la revendication 1, caractérisés en ce que Z représente l'oxygène et $R_1$ l'hydrogène.

3. Composés de formule I selon la revendication 1, caractérisés en ce que $R_1$ représente l'hydrogène, $R_2$ et $R_3$ représentent des groupes alkyle en C1-C4 ou alcoxy en C1-C4 et Q, Z et E ont les significations indiquées en référence à la formule I.

4. Composés de formule I selon la revendication 1, caractérisés en ce que $R_1$ représente l'hydrogène, $R_2$ et $R_3$ représentent des groupes alkyle en C1-C3 ou alcoxy en C1-C3, Z représente l'oxygène, E représente l'azote ou CH, Q représente un groupe hétérocyclique défini en référence à la formule I, éventuellement substitué par le chlore, le brome, le fluor, des groupes nitro, cyano, (alkyle en C1-C4)-carbonyle, (alkyloxy en C1-C4)-carbonyle, alcoxy en C1-C3, alkylthio en C1-C3, alkylsulfonyle en C1-C3, méthoxypropyle, alkylsulfonyle en C1-C3, alkyle en C1-C4, trifluorométhyle, trichlorométhyle, cyano-(alkyle en C1-C4), alcényloxy en C2-C4, alcynyloxy en C3-C4 ou N-(alkyle en C1-C4)-carbonylamino.

5. La N-[1-(3-méthoxy-1-propyl)-imidazole-2-ylsulfonyl]-N'(4-méthoxy-6-méthyl-pyrimidine-2-yl)-urée selon la revendication 1.

6. Procédé de préparation des composés de formule I selon la revendication 1, caractérisé en ce que l'on

fait réagir un dérivé phénylsulfonylé de formule II

$$Q - SO_2W \qquad (II)$$

avec un composé de formule III

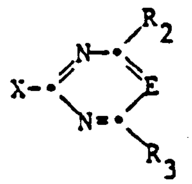

(III)

dans lesquelles X et W représentent l'un des groupes $-NH_2$, $-N = C = Z$ ou $-NR_1-CZ-OR$; les symboles $R_1$, $R_2$, $R_3$, Q, Z et E ont les significations indiquées en référence à la formule I, R représente un groupe aliphatique ou aromatique; sous réserve que les réactifs de formules II et III sont toujours choisis de manière qu'une fonction amino réagisse soit avec un groupe isocyanato ou isothiocyanato, soit avec un groupe $[-NR_1-CZ-OR]$.

7. Procédé selon la revendication 6, caractérisé en ce que R représente un groupe alkyle en C1-C4, phényle ou benzyle.

8. Procédé selon la revendication 6, caractérisé en ce que l'on opère en présence d'un solvant ou mélange solvant inerte dans la réaction.

9. Procédé selon la revendication 8, caractérisé en ce que la réaction est effectuée en présence d'une base.

10. Procédé selon la revendication 8, caractérisé en ce que la réaction est effectuée à des températures allant de -20 à +120°.

11. Produit herbicide et/ou régulateur de la croissance des végétaux, caractérisé en ce qu'il contient au moins un composant actif consistant en un composé de formule I.

12. Produit selon la revendication 11, caractérisé en ce qu'il contient de 0,1 à 95 % en poids d'un composé de formule I selon la revendication 1 et de 1 à 99,9 % en poids d'additifs parmi lesquels 0,1 à 25 % en poids d'un agent tensioactif.

13. Utilisation d'un composé de formule I selon la revendication 1 pour la lutte contre les végétaux indésirables dans les cultures de végétaux utiles et/ou pour la régulation de la croissance des végétaux utiles.

14. Utilisation selon la revendication 13, pour la lutte sélective contre les mauvaises herbes.

15. Utilisation selon la revendication 13, pour l'inhibition de la croissance des végétaux.

16. Procédé pour combattre les mauvaises herbes en pré-levée ou en post-levée et/ou pour agir sur la croissance des végétaux utiles, caractérisé en ce que l'on applique une quantité efficace d'une substance active de formule I selon la revendication 1 sur les végétaux nuisibles ou utiles, des parties de ces végétaux ou leur habitat.

17. Composés de formule

$$Q\text{-}SO_2\text{-}NH\text{-}CZ\text{-}OR$$

dans laquelle Q et Z ont les significations indiquées en référence à la formule I dans la revendication 1 et R représente un groupe aliphatique ou aromatique.

18. Composés selon la revendication 17, caractérisé en ce que R représente un groupe alkyle en C1-C4, phényle ou benzyle.

**Revendications pour l'Etat contractant suivant : AT**

1. Produit herbicide et/ou régulateur de la croissance des végétaux, caractérisé en ce qu'il contient au moins un composant actif consistant en un composé de formule I

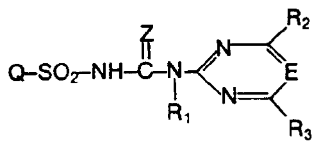

(I)

ou en un seul d'un tel composé,

les symboles de la formule I ayant les significations suivantes:

$R_1$ représente l'hydrogène ou un groupe alkyle en $C_1$-$C_5$,

$R_2$ et $R_3$ représentent chacun, indépendamment l'un de l'autre, l'hydrogène, un groupe alkyle en $C_1$-$C_5$, alcoxy en $C_1$-$C_5$, alkylthio en $C_1$-$C_5$, halogénoalkyle en $C_1$-$C_5$, un halogène, un groupe halogènoalcoxy en $C_1$-$C_5$, halogènoalkylthio en $C_1$-$C_5$, alkylamino en $C_1$-$C_5$, di-(alkyle en $C_1$-$C_4$)-amino, un groupe alcoxyalkyle ou alcoxyalcoxy contenant au maximum 6 atomes de carbone;

Z représente l'oxygène ou le soufre;

E représente -CH= ou -N=, et

Q représente un groupe hétérocyclique à 5 chaînons éventuellement substitué, relié par l'intermédiaire d'un atome de carbone, choisi dans la classe du pyrazole, de l'imidazole, du 1,2,4-triazole, du 1,2,3-triazole, de l'oxazole, du thiazole, de l'isoxazole, de l'isothiazole, du furazanne, du 1,2,4-thiadiazole, du 1,2,4-oxadiazole, du 1,3,4-oxadiazole, du 1,2,3-thiadiazole et du 1,3,4-thiadiazole, les substituants étant choisis parmi les halogènes, les pseudo-halogènes, les groupes nitro, alkyle, halogènoalkyle, alcoxy, alkylthio, hydroxy, halogénoalcoxy, halogénoalkylthio, amino, alkylamino, dialkylamino, alkylcarbonylamino, alkylcarbonyle, alcoxycarbonyle, méthoxypropyle, alkylthiocarbonyle, carbamoyle, alkylaminocarbonyle, dialkylaminocarbonyle, alkylsulfinyle, alkylsulfonyle, alcényloxy, alcynyloxy ou un groupe phényle, phénoxy, phénylthio éventuellement substitué par des groupes alkylcarbonyle, alcoxycarbonyle, des halogènes, des groupes nitro, cyano, alkyle, alcoxy, halogénoalkyle ou halogénoalcoxy, ou un groupe benzyle éventuellement substitué par des halogènes et/ou des groupes alkyle.

2. Produit selon la revendication 1, caractérisé en ce que Z représente l'oxygène et $R_1$ l'hydrogène.

3. Produit selon la revendication 1, caractérisé en ce que $R_1$ représente l'hydrogène, $R_2$ et $R_3$ représentent des groupes alkyle en $C_1$-$C_4$ ou alcoxy en $C_1$-$C_4$ et Q, Z et E ont les significations indiquées en références à la formule I.

4. Produit selon la revendication 1, caractérisé en ce que $R_1$ représente l'hydrogène, $R_2$ et $R_3$ représentent des groupes alkyle en $C_1$-$C_3$ ou alcoxy en $C_1$-$C_3$, Z représente l'oxygène, E représente l'azote ou CH, Q représente un groupe hétérocyclique défini en référence à la formule I, qui peut le cas échéant être substitué par le chlore, le brome, le fluor, des groupes nitro, cyano, (alkyle en $C_1$-$C_4$)-carbonyle, (alkyloxy en $C_1$-$C_4$)-carbonyle, alcoxy en $C_1$-$C_3$, alkylthio en $C_1$-$C_3$, alkylsulfonyle en $C_1$-$C_3$, méthoxypropyle, alkylsulfonyle en $C_1$-$C_3$, alkyle en $C_1$-$C_4$, trifluorométhyle trichlorométhyle, cyano-(alkyle en $C_1$-$C_4$), alcényloxy en $C_2$-$C_4$, alcynyloxy en $C_3$-$C_4$ ou N-(alkyle en $C_1$-$C_4$)-carbonylamino.

5. Produit selon la revendication 1, caractérisé en ce qu'il contient en tant que substance active la N-[1-(3-méthoxy-1-propyl)-imidazole-2-ylsulfonyl]-N'-(4-méthoxy-6-méthyl-pyrimidine-2-yl)-urée.

6. Procédé de préparation des composés de formule I selon la revendication 1, caractérisé en ce que l'on fait réagir un dérivé phénylsulfonylé de formule II

$$Q\text{-}SO_2W \qquad (II)$$

avec un composé de formule III

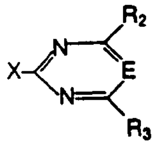

(III)

dans lesquelles X et W représentent l'un des groupes $-NH_2$, -N = C = Z ou $-NR_1$-CZ-OR; les symboles $R_1$, $R_2$, $R_3$, Q, Z et E ont les significations indiquées en référence à la formule I, R représente un groupe aliphatique ou aromatique; sous réserve que les réactifs de formules II et III sont toujours choisis de manière qu'une fonction amino réagisse soit avec un groupe isocyanato ou isothiocyanato, soit avec un groupe [-$NR_1$-CZ-OR].

7. Procédé selon la revendication 6, caractérisé en ce que R représente un groupe alkyle en $C_1$-$C_4$, phényle ou benzyle.

8. Procédé selon la revendication 6, caractérisé en ce que l'on opère en présence d'un solvant ou mélange solvant inerte dans la réaction.

9. Procédé selon la revendication 8, caractérisé en ce que l'on effectue la réaction en présence d'une base.

10. Procédé selon la revendication 8, caractérisé en ce que l'on effectue la réaction à des températures de - 20 à + 120°.

11. Produits selon la revendication 1, caractérisé en ce qu'ils contiennent de 0,1 à 95% en poids d'un composé de formule I selon la revendication 1 et de 1 à 99,9% en poids d'additifs parmi lesquels 0,1 à 25% en poids d'un agent tensioactif.

12. Utilisation d'un composé de formule I selon la revendication 1 pour la lutte contre les végétaux indésirables dans les cultures de végétaux utiles et/ou pour la régulation de la croissance des végétaux utiles.

13. Utilisation selon la revendication 12, pour la lutte sélective contre les mauvaises herbes.

14. Utilisation selon la revendication 12, pour l'inhibition de la croissance des végétaux.

15. Procédé pour combattre les mauvaises herbes en pré-levée ou en post-levée et/ou pour agir sur la croissance des végétaux utiles, caractérisé en ce que l'on applique une quantité efficace d'une substance active de formule I selon la revendication 1 sur les végétaux nuisibles ou utiles, des parties de ces végétaux ou leur habitat.

**Revendications pour l'Etat contractant suivant : BE**

1. Composés de formule I

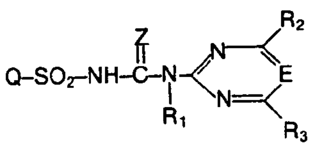

(I)

dans laquelle

$R_1$ représente l'hydrogène ou un groupe alkyle en $C_1$-$C_5$,

$R_2$ et $R_3$ représentent chacun, indépendamment l'un de l'autre, l'hydrogène, un groupe alkyle en $C_1$-$C_5$, alcoxy en $C_1$-$C_5$, alkylthio en $C_1$-$C_5$, halogénoalkyle en $C_1$-$C_5$, un halogène, un groupe halogénoalcoxy en $C_1$-$C_5$, halogénoalkylthio en $C_1$-$C_5$, alkylamino en $C_1$-$C_5$, di-(alkyle en $C_1$-$C_4$)-amino, un groupe alcoxyalkyle ou alcoxyalcoxy contenant au maximum 6 atomes de carbone;

Z représente l'oxygène ou le soufre;

E représente -CH= ou -N=, et

Q représente un groupe hétérocyclique à 5 chaînons éventuellement substitué, relié par l'intermédiaire d'un atome de carbone, choisi dans la classe du pyrazole, de l'imidazole, du 1,2,4-triazole, du 1,2,3-triazole, de l'oxazole, du thiazole, de l'isoxazole, de l'isothiazole, du furazanne, du 1,2,4-thiadiazole, du 1,2,4-oxadiazole, du 1,3,4-oxadiazole, du 1,2,3-thiadiazole et du 1,3,4-thiadiazole, les substituants étant choisis parmi les halogènes, les pseudo-halogènes, les groupes nitro, alkyle, halogénoalkyle, alcoxy, alkylthio, hydroxy, halogénoalcoxy, halogénoalkylthio, amino, alkylamino, dialkylamino, alkylcarbonylamino, alkylcarbonyle, alcoxycarbonyle, méthoxypropyle, alkylthiocarbonyle, carbamoyle, alkylaminocarbonyle, dialkylaminocarbonyle, alkylsulfinyle, alkylsulfonyle, alcényloxy, alcynyloxy ou un groupe phényle, phénoxy, phénylthio éventuellement substitué par des groupes alkylcarbonyle, alcoxycarbonyle, des halogènes, des groupes nitro, cyano, alkyle, alcoxy, halogènoalkyle ou halogénoalcoxy, ou un groupe benzyle éventuellement substitué par des halogènes et/ou des groupes alkyle,

y compris leurs sels.

**2.** Composés de formule I selon la revendication 1, caractérisés en ce que Z représente l'oxygène et $R_1$ l'hydrogène.

**3.** Composés de formule I selon la revendication 1, caractérisés en ce que $R_1$ représente l'hydrogène, $R_2$ et $R_3$ représentent des groupes alkyle en $C_1$-$C_4$ ou alcoxy en $C_1$-$C_4$ et Q, Z et E ont les significations indiquées en référence à la formule I.

**4.** Composés de formule I selon la revendication 1, caractérisés en ce que $R_1$ représente l'hydrogène, $R_2$ et $R_3$ représentent des groupes alkyle en $C_1$-$C_3$ ou alcoxy en $C_1$-$C_3$, Z représente l'oxygène, E représente l'azote ou CH, Q représente un groupe hétérocyclique défini en référence à la formule I, éventuellement substitué par le chlore, le brome, le fluor, des groupes nitro, cyano, (alkyle en $C_1$-$C_4$)-carbonyle, (alkyloxy en $C_1$-$C_4$)-carbonyle, alcoxy en $C_1$-$C_3$, alkylthio en $C_1$-$C_3$, alkylsulfonyle en $C_1$-$C_3$, méthoxypropyle, alkylsulfonyle en $C_1$-$C_3$, alkyle en $C_1$-$C_4$, trifluorométhyle, trichlorométhyle, cyano-(alkyle en $C_1$-$C_4$), alcényloxy en $C_2$-$C_4$, alcynyloxy en $C_3$-$C_4$ ou N-(alkyle en $C_1$-$C_4$)-carbonylamino.

**5.** La N-[1-(3-méthoxy-1-propyl)-imidazole-2-ylsulfonyl]-N'-(4-méthoxy-6-méthyl-pyrimidine-2-yl)-urée selon la revendication 1.

**6.** Procédé de préparation des composés de formule I selon la revendication 1, caractérisé en ce que l'on fait réagir un dérivé phénylsulfonylé de formule II

$$Q\text{-}SO_2W \qquad (II)$$

avec un composé de formule III

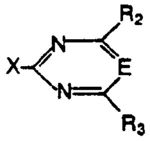

(III)

dans lesquelles X et W représentent l'un des groupes $-NH_2$, -N=C=Z ou $-NR_1$-CZ-PR; les symboles $R_1$, $R_2$, $R_3$, Q, Z et E ont les significations indiquées en référence à la formule I, R représente un groupe aliphatique ou aromatique; sous réserve que les réactifs de formules II et III sont toujours choisis de manière qu'une fonction amino réagisse soit avec un groupe isocyanato ou isothiocyanato, soit avec un groupe [-$NR_1$-CZ-OR].

**7.** Procédé selon la revendication 6, caractérisé en ce que R représente un groupe alkyle en $C_1$-$C_4$, phényle ou benzyle.

**8.** Procédé selon la revendication 6, caractérisé en ce que l'on opère en présence d'un solvant ou mélange solvant inerte dans la réaction.

**9.** Procédé selon la revendication 8, caractérisé en ce que la réaction est effectuée en présence d'une base.

**10.** Procédé selon la revendication 8, caractérisé en ce que la réaction est effectuée à des températures allant de -20 à + 120°.

**11.** Produit herbicide et/ou régulateur de la croissance des végétaux, caractérisé en ce qu'il contient au moins un composant actif consistant en un composé de formule I.

**12.** Produit selon la revendication 11, caractérisé en ce qu'il contient de 0,1 à 95% en poids d'un composé de formule I selon la revendication 1 et 1 à 99,9% en poids d'additifs parmi lesquels 0,1 à 25% en poids d'un agent tensioactif.

**13.** Utilisation d'un composé de formule I selon la revendication 1 pour la lutte contre les végétaux indésirables dans les cultures de végétaux utiles et/ou pour la régulation de la croissance des végétaux utiles.

**14.** Utilisation selon la revendication 13, pour la lutte sélective contre les mauvaises herbes.

**15.** Utilisation selon la revendication 13, pour l'inhibition de la croissance des végétaux.

**16.** Procédé pour combattre les mauvaises herbes en pré-levée ou en post-levée et/ou pour agir sur la croissance des végétaux utils, caractérisé en ce que l'on applique une quantité efficace d'une substance active de formule I selon la revendication 1 sur les végétaux nuisibles ou utiles, des parties de ces végétaux ou leur habitat.

**17.** Composés de formule

$$Q\text{-}SO_2\text{-}NH\text{-}CZ\text{-}OR$$

dans laquelle Q et Z ont les significations indiquées en référence à la formule I dans la revendication 1 et R représente un groupe aliphatique ou aromatique.

**18.** Composés selon la revendication 17, caractérisé en ce que R représente un groupe alkyle en $C_1$-$C_4$, phényle ou benzyle.